# EUROPÄISCHE PATENTSCHRIFT

(11) **EP 2 872 589 B1**
(45) Veröffentlichungstag und Bekanntmachung des Hinweises auf die Patenterteilung: **26.07.2017**
(21) Anmeldenummer: 13728121.8
(22) Anmeldetag: 12.06.2013
(51) Int. Cl.: C09K 11/06, C07D 471/00, H05B 33/20, C07D 471/06, C07D 471/16, C07D 487/06, C07D 487/16, C07D 498/06, C07D 498/16, C07D 513/06, C07D 513/16, C07D 519/00, H01L 51/00, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX CONÇUS POUR DES DISPOSITIFS ÉLECTROLUMINESCENTS ORGANIQUES

(30) Priorität: 10.07.2012 EP 12005099
(43) Veröffentlichungstag der Anmeldung: 20.05.2015
(73) Patentinhaber: Merck Patent GmbH, 64293 Darmstadt (DE)
(72) Erfinder: PARHAM, Amir Hossain, 60486 Frankfurt am Main (DE); PFLUMM, Christof, 64291 Darmstadt (DE); JATSCH, Anja, 60489 Frankfurt am Main (DE); EBERLE, Thomas, 76829 Landau (DE); KROEBER, Jonas Valentin, 60311 Frankfurt am Main (DE)
(86) Internationale Anmeldenummer: PCT/EP2013/001724
(87) Internationale Veröffentlichungsnummer: WO 2014/008967

(56) Entgegenhaltungen:
- EP-A2- 2 182 040
- WO-A1-2006/033563
- WO-A1-2008/066358
- DE-A1-102009 023 155
- JP-A- H05 107 784
- JP-A- H11 339 868

## Beschreibung

Die vorliegende Erfindung betrifft Materialien für die Verwendung in elektronischen Vorrichtungen, insbesondere in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Dies gilt insbesondere für OLEDs, welche im kürzerwelligen Bereich emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial.

Überraschend wurde gefunden, dass bestimmte, unten näher beschriebene Verbindungen diese Aufgabe lösen und zu Verbesserungen der organischen Elektrolumineszenzvorrichtung führen, insbesondere hinsichtlich der Lebensdauer, der Effizienz und/oder der Betriebsspannung. Dies gilt insbesondere bei Einsatz der erfindungsgemäßen Verbindungen als Matrixmaterial, aber auch bei Einsatz als Lochtransportmaterial oder Lochinjektionsmaterial. Diese Materialien sowie organische Elektrolumineszenzvorrichtungen, welche derartige Verbindungen enthalten, sind daher der Gegenstand der vorliegenden Erfindung.

Aus der WO 2011/042107 sind überbrückte Carbazolderivate bekannt, welche mit Triazin oder anderen elektronenarmen Heteroarylgruppen substituiert sind, wobei die Substitution mit den elektronenarmen Heteroarylgruppen wesentlich ist. Diese Verbindungen werden insbesondere als Matrixmaterialien für phosphoreszierende Emitter beschrieben. Verbindungen ohne dieses Substitutionsmuster werden nicht offenbart.

Aus der WO 2011/088877 sind überbrückte Carbazolderivate bekannt, welche mit Diarylamino-, Triarylamino- oder Carbazolgruppen substituiert sind, wobei die Substitution mit diesen Gruppen wesentlich ist. Verbindungen ohne dieses Substitutionsmuster werden nicht offenbart.

Es hat sich überraschend gezeigt, dass der Einsatz der erfindungsgemäßen Verbindungen in organischen Elektrolumineszenzvorrichtungen zu guten elektronischen Eigenschaften führt.

Gegenstand der vorliegenden Erfindung ist daher eine Verbindung gemäß der folgenden Formel (1 a) oder (2a), 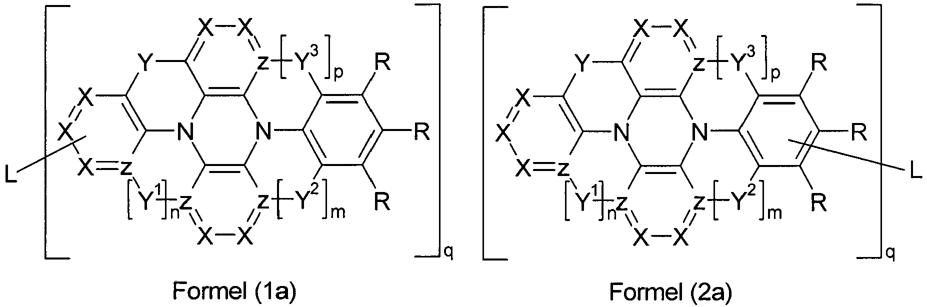 wobei für die verwendeten Symbole und Indizes gilt:
- X: ist bei jedem Auftreten gleich oder verschieden CR oder N; bzw. X steht für C, wenn an diese Gruppe X eine Gruppe L gebunden ist;
- Y, Y¹, Y², Y³: ist bei jedem Auftreten gleich oder verschieden eine Einfachbindung oder C(R¹)₂, O oder S; mit der Maßgabe, dass Y und Y¹ nicht gleichzeitig für eine Einfachbindung stehen und dass Y² und Y³ nicht gleichzeitig für eine Einfachbindung stehen;
- Z: ist bei jedem Auftreten gleich oder verschieden CR oder N; bzw. Z steht für C, wenn an diese Gruppe Z eine Gruppe Y¹ bzw. Y² bzw. Y³ gebunden ist;
- L: ist gleich oder verschieden R, wenn q = 1 ist, oder ist eine bi-, tri-, tetra-, penta- oder hexavalente geradkettige Alkylen-, Alkyliden-, Alkylenoxy- oder Thioalkylenoxygruppe mit 1 bis 40 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R² substituiert sein kann, wobei jeweils eine oder mehrere nicht benachbarte CH₂-Gruppen durch -R²C=CR²-, -C=C-, Si(R²)₂, C=O, C=NR², P(=O)R², S=O, SO₂, -O-, -S- oder -CONR²- ersetzt sein können und wobei ein oder mehrere H-Atome durch D, F, Cl, Br, I, CN oder NO₂ ersetzt sein können, oder ein bi-, tri-, tetra-, penta- oder hexavalentes aromatisches Ringsystem mit 5 bis 40, aromatischen Ringatomen, welches durch einen oder mehrere Reste R² substituiert sein kann, oder heteroaromatisches Ringsystem mit 5 bis 60 aromatischen Ringatomen, welches als Heteroarylgruppen ausschließlich schwefelhaltige oder sauerstoffhaltige Heteroarylgruppen enthält und welches durch einen oder mehrere Reste R² substituiert sein kann, oder P(R²)₃₋ᵣ, P(=O)(R²)₃₋ᵣ, C(R²)₄₋ᵣ, Si(R²)₄₋ᵣ, N(Ar)₃₋ᵣ, wobei r für 2, 3 oder 4 steht, mit der Maßgabe, dass r nicht größer ist als die maximale Valenz von L; oder L ist eine chemische Bindung, wobei dann q = 2 ist; dabei ist die Valenz der Gruppe L = q + 1;
- n, m, p: ist bei jedem Auftreten gleich oder verschieden 0 oder 1, wobei n = 0 bzw. m = 0 bzw. p = 0 bedeutet, dass die entsprechende Gruppe Y¹ bzw. Y² bzw. Y³ nicht vorhanden ist und dass statt Y² bzw. Y³ an dem entsprechenden Kohlenstoffatom eine Gruppe R gebunden ist;
- q: ist 1, 2, 3, 4, 5 oder 6;
- R, R¹: ist bei jedem Auftreten gleich oder verschieden ausgewählt aus der Gruppe bestehend aus H, D, F, Cl, Br, I, CN, NO₂, C(=O)Ar, C(=O)R², P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C=C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen Ringsystem mit 6 bis 60 aromatischen Ringatomen, das jeweils mit einem oder mehreren Resten R² substituiert sein kann, einem heteroaromatischen Ringsystem mit 5 bis 60 aromatischen Ringatomen, welches als Heteroarylgruppen schwefelhaltige oder sauerstoffhaltige Heteroarylgruppen enthält und welches mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Aralkyl- oder Heteroaralkylgruppe mit 5 bis 60 aromatischen Ringatomen, die mit einem oder mehreren Resten R² substituiert sein kann, wobei optional zwei Substituenten R¹, die in derselben Gruppe Y gebunden sind, miteinander ein mono- oder polycyclisches, aliphatisches, aromatisches oder heteroaromatisches Ringsystem bilden können, das mit einem oder mehreren Resten R² substituiert sein kann; weiterhin können zwei benachbarte Reste R einen ankondensierten Benzoring bilden, der mit einem oder mehreren Resten R² substituiert sein kann; dabei ist die Gruppe R in Formel (2) nicht vorhanden, wenn an das entsprechende Kohlenstoffatom die Gruppe L bindet;
- R²: ist bei jedem Auftreten gleich oder verschieden ausgewählt aus der Gruppe bestehend aus H, D, F, Cl, Br, I, CN, NO₂, C(=O)Ar, C(=O)R³, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder eine Aralkyl- oder Heteroaralkylgruppe mit 5 bis 60 aromatischen Ringatomen;
- Ar: ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar, welche an dasselbe P-Atom binden, auch durch eine Einfachbindung oder eine Brücke, ausgewählt aus N(R³), C(R³)₂, O oder S, miteinander verbrückt sein;
- R³: ist ausgewählt aus der Gruppe bestehend aus H, D, F, CN, einem aliphatischem Kohlenwasserstoffrest mit 1 bis 20 C-Atomen oder einem aromatischem Ringsystem mit 5 bis 30 aromatischen Ringatomen, in dem ein oder mehrere H-Atome durch D, F, Cl, Br, I oder CN ersetzt sein können;
dabei sind die folgenden Verbindungen von der Erfindung ausgenommen:

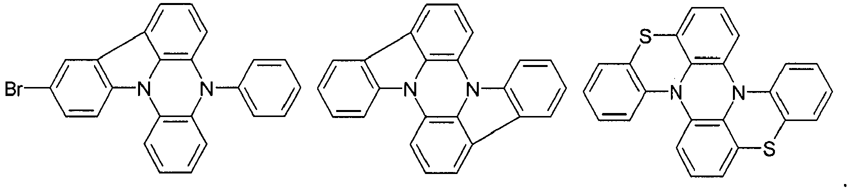

Unter benachbarten Resten R werden Reste R verstanden, die an Kohlenstoffatome binden, die direkt miteinander verknüpft sind.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus O und/oder S und gegebenenfalls zusätzlich N. Dabei wird unter einer Arylgruppe bzw. Heteroarylgruppe entweder ein einfacher aromatischer Cyclus, also Benzol, bzw. ein einfacher heteroaromatischer Cyclus, beispielsweise Thiophen, Furan, etc., oder eine kondensierte (anellierte) Aryl- oder Heteroarylgruppe, beispielsweise Naphthalin, Anthracen, Phenanthren, Benzofuran, Benzothiophen, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Schwefelhaltige oder sauerstoffhaltige Heteroarylgruppen im Sinne der vorliegenden Erfindung sind Heteroarylgruppen, welche mindestens ein Schwefelatom oder Sauerstoffatom enthalten, insbesondere Furan, Thiophen, Benzofuran, Benzothiophen, Dibenzofuran und Dibenzothiophen.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus O und/oder S und gegebenenfalls zusätzlich N. Dabei sind die im heteroaromatischen Ringsystem vorhandenen Heteroarylgruppen ausschließlich schwefelhaltige oder sauerstoffhaltige Gruppen. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch ein oder mehrere C-Atome verbunden sein können. So sollen beispielsweise auch Systeme wie Fluoren, 9,9'-Spirobifluoren, 9,9-Diarylfluoren, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten R² oder R³ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Phenothiazin, Phenoxazin, Oxazol, Benzoxazol, Naphthoxazol, Anthroxazol, Phenanthroxazol, Isoxazol, 1,2-Thiazol, 1,3-Thiazol, Benzothiazol, Phenothiazin, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol und Benzothiadiazol oder Gruppen, die abgeleitet sind von Kombination dieser Systeme.

In einer bevorzugten Ausführungsform der Erfindung steht Y, Y¹, Y² und Y³ gleich oder verschieden bei jedem Auftreten für eine Einfachbindung oder C(R¹)₂.

In einer weiteren bevorzugten Ausführungsform der Erfindung steht maximal eine der Gruppen X und Z pro Cyclus für N und die anderen Gruppen X und Z in diesem Cyclus stehen für CR, bzw. Z steht für C, wenn an dieses Z eine Gruppe Y¹ bzw. Y² bzw. Y³ gebunden ist, und X steht für C, wenn an dieses eine Gruppe L gebunden ist. Besonders bevorzugt stehen alle X und Z für CR, bzw. Z steht für C, wenn an dieses Z eine Gruppe Y¹ bzw. Y² bzw. Y³ gebunden ist, und X steht für C, wenn an dieses X eine Gruppe L gebunden ist.

Eine besonders bevorzugte Ausführungsform der Formel (1) bzw. (2) ist eine Verbindung der folgenden Formel (1b) bzw. (2b), 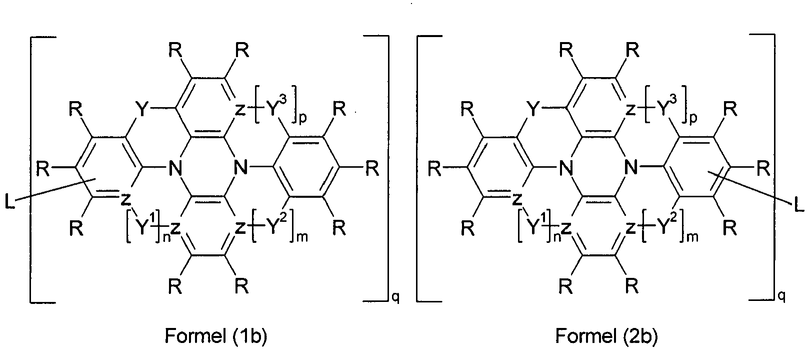 wobei für die verwendeten Symbole gilt:
- Y, Y¹, Y², Y³: ist gleich oder verschieden bei jedem Auftreten eine Einfachbindung, C(R¹)₂, NR¹, O oder S, insbesondere eine Einfachbindung oder C(R¹)2;
- Z: ist CR, bzw. Z ist C, wenn an dieses Z eine Gruppe Y¹ bzw. Y² bzw. Y³ gebunden ist;
und die weiteren verwendeten Symbole und Indizes die oben genannten Bedeutungen aufweisen;
dabei ist der Rest R nicht vorhanden, wenn in dieser Position eine Gruppe L gebunden ist.

Bevorzugt sind weiterhin Verbindungen der Formel (1a), (1b) bzw. (2a), (2b), in denen Y für eine Einfachbindung steht. Ebenfalls bevorzugt sind Verbindungen der Formel (1 a), (1 b) bzw. (2a), (2b), in denen Y für C(R¹)₂, O oder S steht und Y² gleich oder verschieden für C(R¹)₂, O oder S steht und n = 0 und p = 0 ist. Ebenfalls bevorzugt sind Verbindungen der Formel (1 a), (1 b) bzw. (2a), (2b), in denen Y für C(R¹)₂, O oder S steht und Y³ gleich oder verschieden für C(R¹)₂, O oder S steht und n = 0 und m = 0 ist.

Bevorzugte Ausführungsformen der Formel (1a) bzw. (1b) sind somit die Verbindungen der folgenden Formeln (3) bis (5) und bevorzugte Ausführungsformen der Formel (2a) bzw. (2b) sind die Verbindungen der folgenden Formeln (6) bis (8), 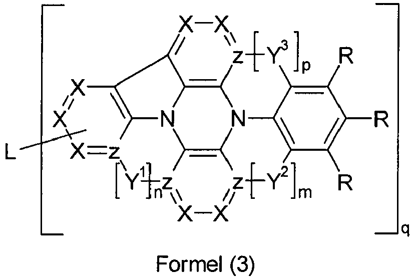 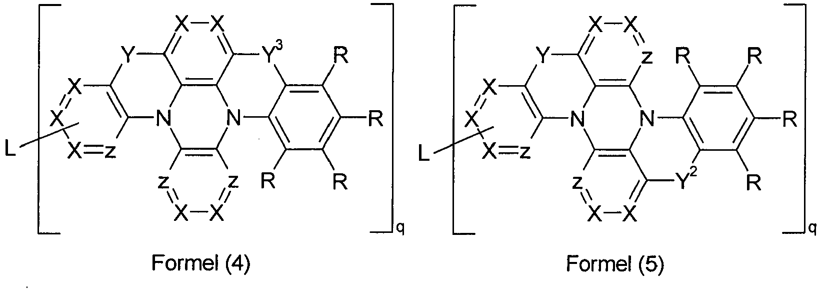 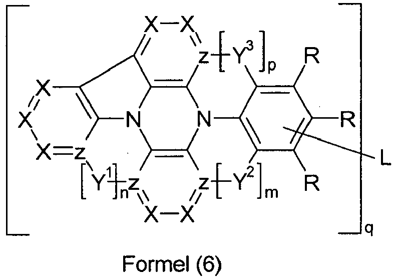 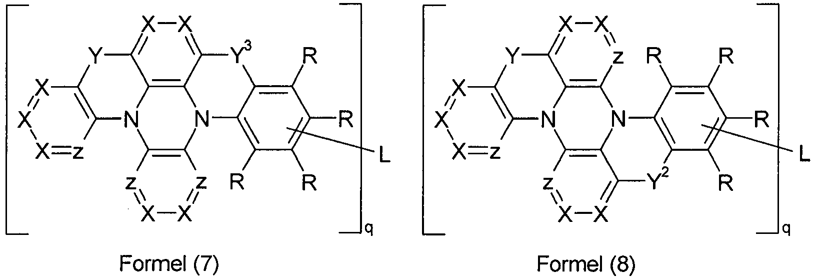 wobei die verwendeten Symbole und Indizes die oben genannten Bedeutungen aufweisen und die Gruppe R nicht vorhanden ist, wenn in dieser Position eine Gruppe L gebunden ist.

In einer bevorzugten Ausführungsform der Verbindungen gemäß Formel (1) bzw. Formel (2) bzw. der oben genannten bevorzugten Ausführungsformen ist der Index q = 1 und L steht für eine Gruppe R.

Bevorzugte Ausführungsformen der Verbindungen gemäß den Formeln (3) bis (8) sind somit die Verbindungen gemäß den folgenden Formeln (3a), (4a) und (5a), 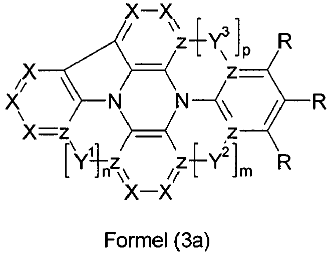 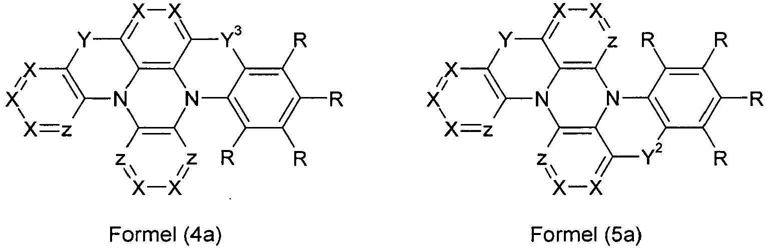 wobei die verwendeten Symbole und Indizes die oben genannten Bedeutungen aufweisen.

Bevorzugte Ausführungsformen der Verbindung der Formel (3) sind die Verbindungen der folgenden Formeln (9) bis (13), und bevorzugte Ausführungsformen der Verbindung der Formel (6) sind die Verbindungen der folgenden Formeln (14) bis (18), 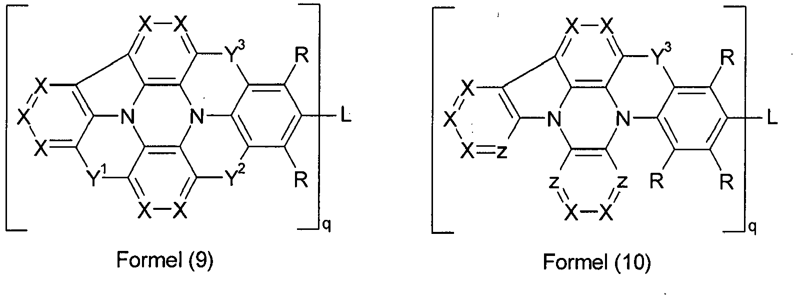 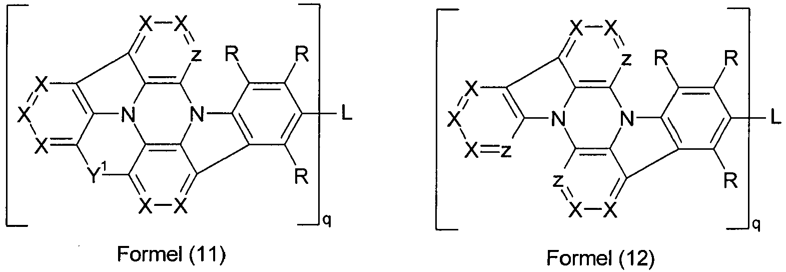 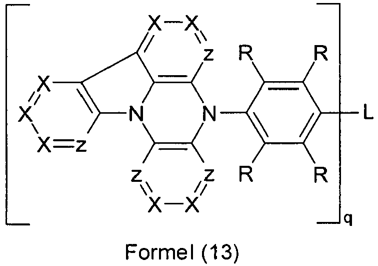 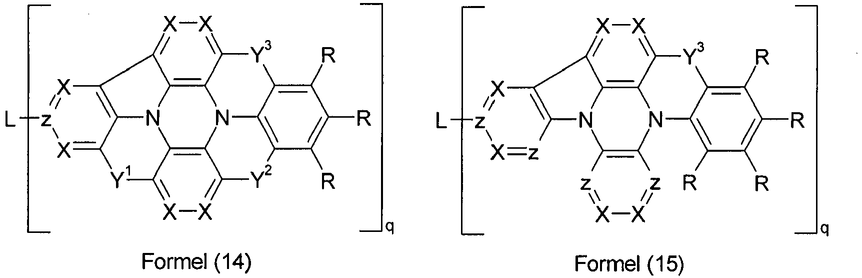 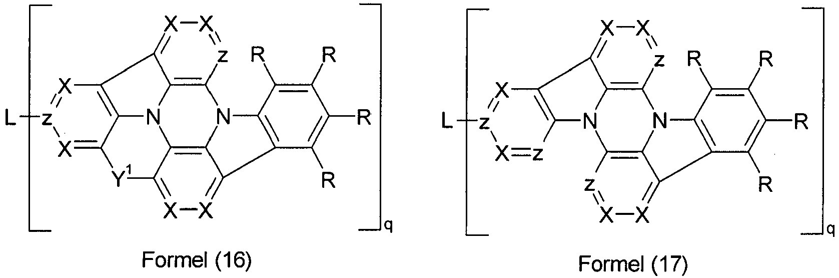 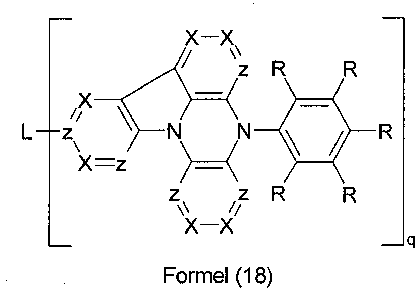 wobei die verwendeten Symbole und Indizes die oben genannten Bedeutungen aufweisen. Dabei steht in Formel (9), (10), (14) und (15) Y³ bevorzugt für eine Einfachbindung.

Bevorzugte Ausführungsformen der Verbindung der Formel (3a) sind die Verbindungen der folgenden Formeln (19) bis (23), 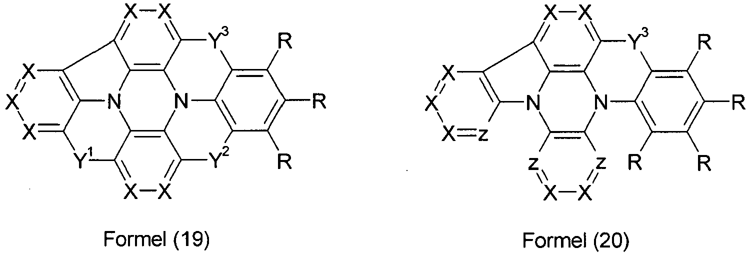 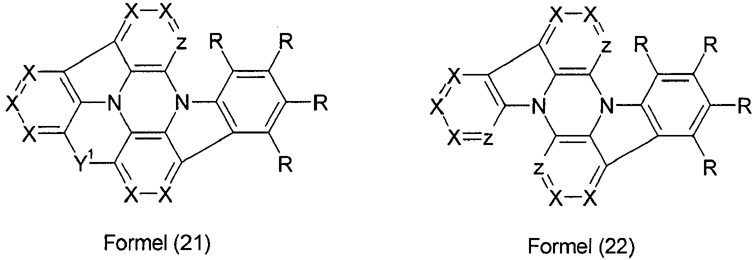 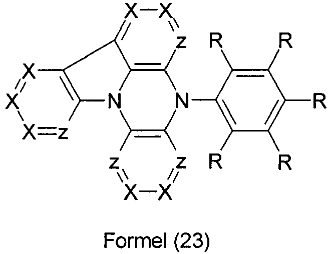 wobei die verwendeten Symbole und Indizes die oben genannten Bedeutungen aufweisen. Dabei steht in Formel (19) und (20) Y³ bevorzugt für eine Einfachbindung.

In einer bevorzugten Ausführungsform der Erfindung stehen in den Verbindungen der Formel (3) bis (23) pro Cyclus insgesamt maximal eine der Gruppen X und Z für N. Besonders bevorzugt stehen alle Gruppen X und Z für CR bzw. C.

Besonders bevorzugte Ausführungsformen der Erfindung sind daher die Verbindungen der folgenden Formeln (19a) bis (23a), 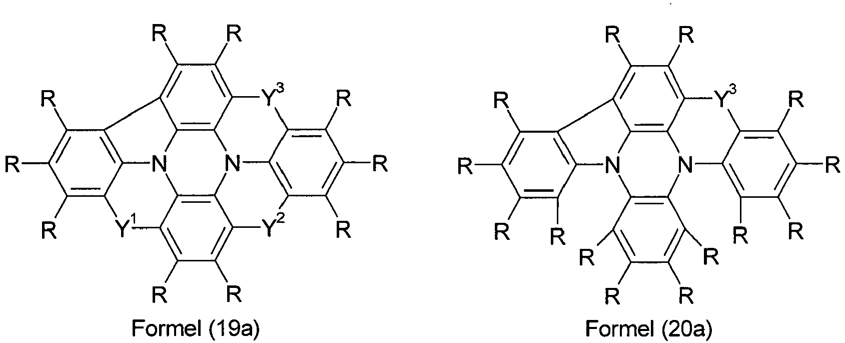 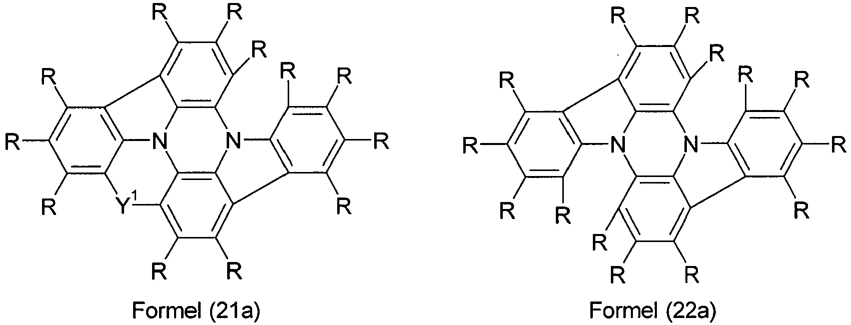 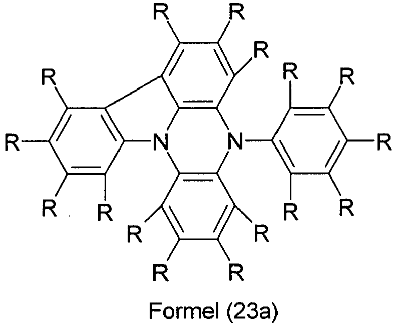 wobei die verwendeten Symbole und Indizes die oben genannten Bedeutungen aufweisen. Dabei steht in Formel (19a) und (20a) Y³ bevorzugt für eine Einfachbindung.

Ganz besonders bevorzugte Ausführungsformen der Erfindung sind daher die Verbindungen der folgenden Formeln (19b) bis (23b), 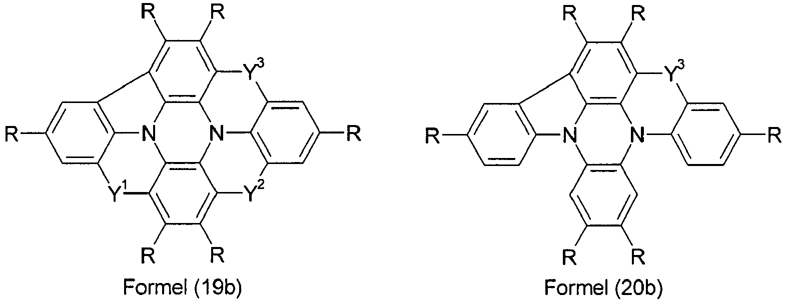 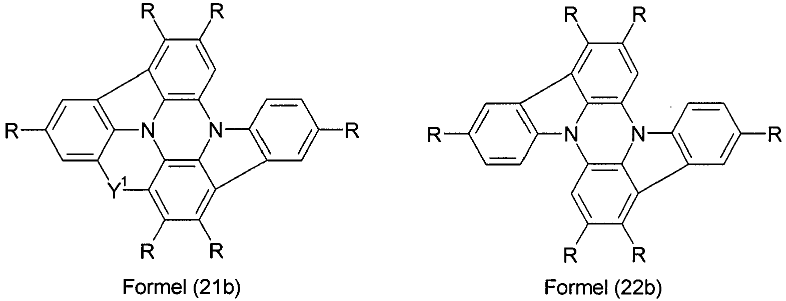 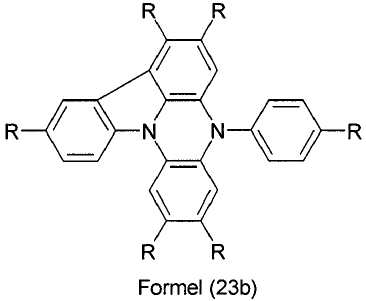 wobei die verwendeten Symbole und Indizes die oben genannten Bedeutungen aufweisen. Dabei steht in Formel (19b) und (20b) Y³ bevorzugt für eine Einfachbindung.

Bevorzugte Ausführungsformen der Formeln (4a) und (5a) sind die Verbindungen der folgenden Formeln (24) und (25), 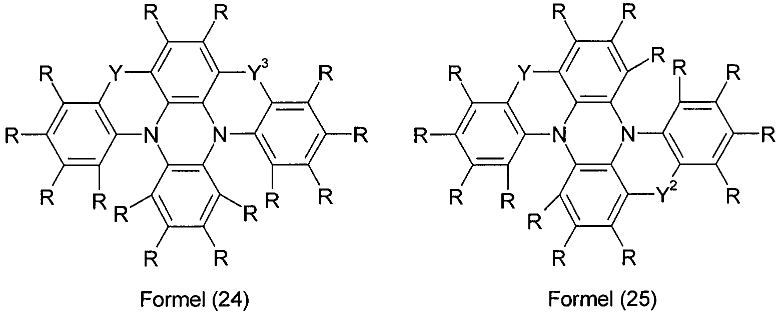 wobei die verwendeten Symbole die oben genannten Bedeutungen aufweisen und Y, Y² und Y³ bevorzugt gleich oder verschieden bei jedem Auftreten für eine Einfachbindung, C(R¹)₂, O oder S steht, insbesondere gleich oder verschieden für eine Einfachbindung oder C(R¹)₂.

Bevorzugte Ausführungsformen der Formeln (24) und (25) sind die Verbindungen der folgenden Formeln (24a) und (25a), 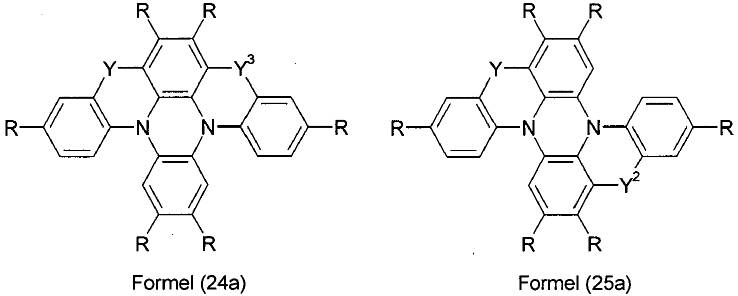 wobei die verwendeten Symbole die oben genannten Bedeutungen auf weisen und Y, Y² und Y³ bevorzugt gleich oder verschieden bei jedem Auftreten für eine Einfachbindung, C(R¹)₂, O oder S steht, insbesondere gleich oder verschieden für eine Einfachbindung oder C(R¹)₂.

Bevorzugte Substituenten R sind gleich oder verschieden bei jedem Auftreten ausgewählt aus der Gruppe bestehend aus H, D, F, CN, C(=O)Ar, C(=O)R², P(=O)(Ar)₂, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, wobei eine oder mehrere nicht-benachbarte CH₂-Gruppen durch O oder S ersetzt sein können und wobei ein oder mehrere H-Atome durch D oder F ersetzt sein können, einem aromatischen Ringsystem mit 6 bis 24 aromatischen Ringatomen, das mit einem oder mehreren Resten R² substituiert sein kann, einem heteroaromatischen Ringsystem mit 5 bis 24 aromatischen Ringatomen, welches als Heteroarylgruppen ausschließlich schwefelhaltige oder sauerstoffhaltige Heteroarylgruppen enthält und welches mit einem oder mehreren Resten R² substituiert sein kann, oder einer Aralkyl- oder Heteroaralkylgruppe mit 5 bis 24 aromatischen Ringatomen, die mit einem oder mehreren Resten R² substituiert sein kann, wobei optional zwei benachbarte Substituenten R miteinander einen ankondensierten Benzoring bilden können, der mit einem oder mehreren Resten R² substituiert sein kann.

Besonders bevorzugte Substituenten R sind gleich oder verschieden bei jedem Auftreten ausgewählt aus der Gruppe bestehend aus H, einer geradkettigen Alkylgruppe mit 1 bis 4 C-Atomen, insbesondere Methyl, oder einer verzweigten oder cyclischen Alkylgruppe mit 3 bis 8 C-Atomen, die jeweils mit einem oder mehreren Resten R² substituiert sein kann, einem aromatischen Ringsystem mit 6 bis 24 aromatischen Ringatomen, das mit einem oder mehreren Resten R² substituiert sein kann, einem heteroaromatischen Ringsystem mit 5 bis 24 aromatischen Ringatomen, welches als Heteroarylgruppen ausschließlich schwefelhaltige oder sauerstoffhaltige Heteroarylgruppen enthält und welches mit einem oder mehreren Resten R² substituiert sein kann.

Wenn R für ein aromatisches bzw. heteroaromatisches Ringsystem steht, dann ist dieses bevorzugt ausgewählt aus den Gruppen der folgenden Formeln (26) bis (58), 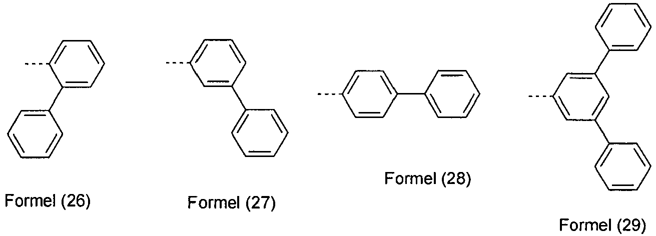 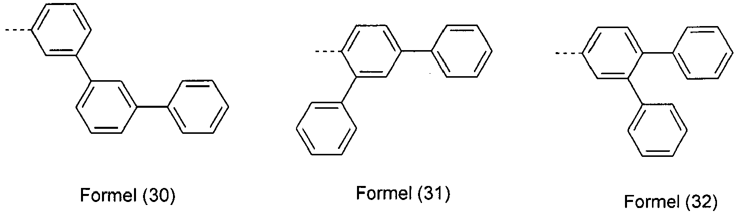 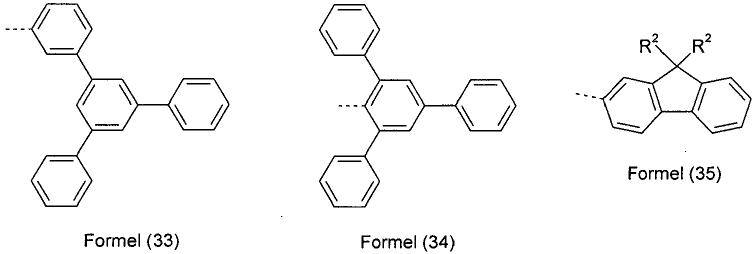 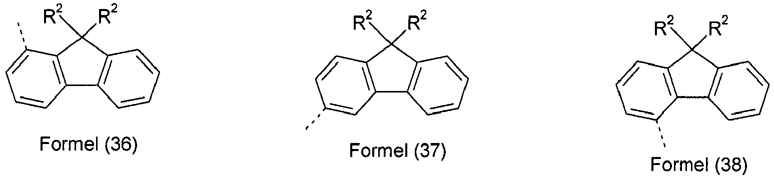 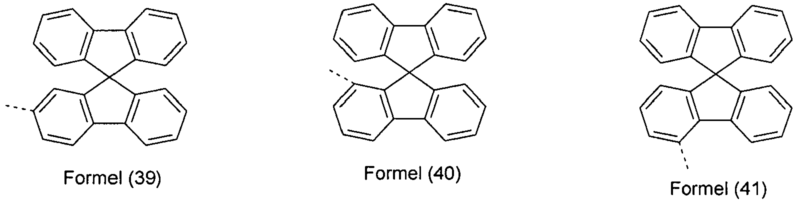 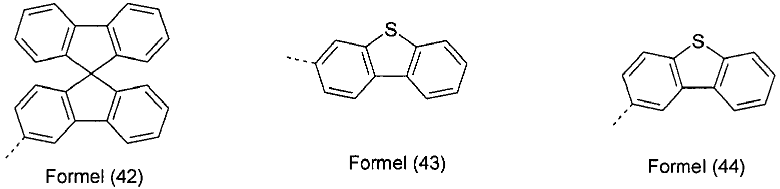 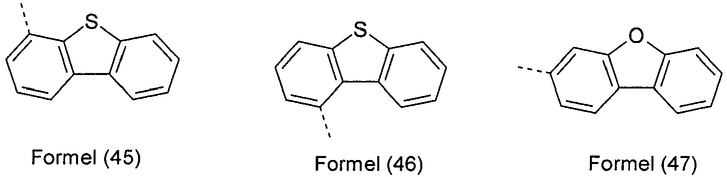 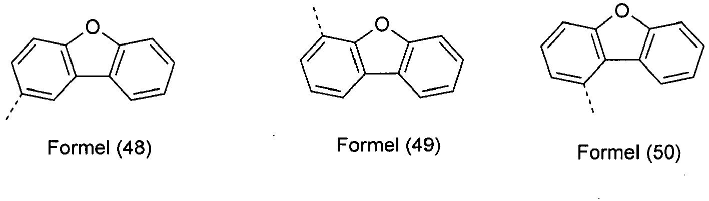 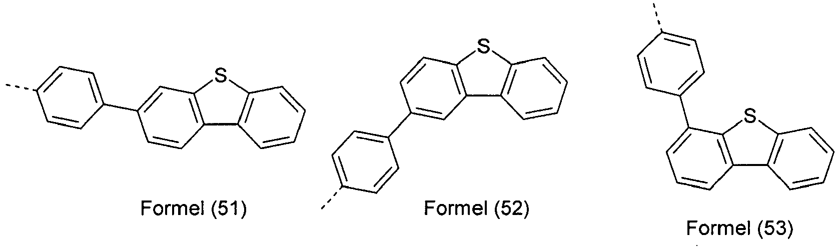 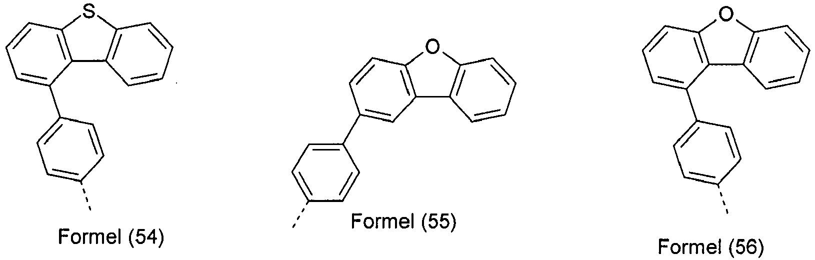 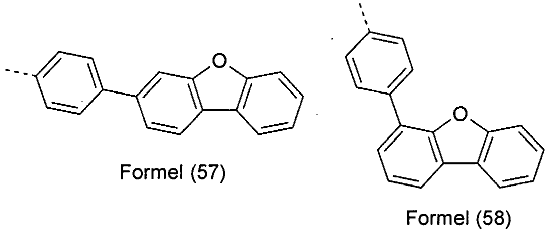 wobei die gestrichelte Bindung die Bindung an das Grundgerüst andeutet und die Gruppen durch einen oder mehrere Reste R² substituiert sein können, bevorzugt aber unsubstituiert sind.

Dabei steht R² in den Gruppen der Formeln (35) bis (38) bevorzugt gleich oder verschieden für eine Alkylgruppe mit 1 bis 10 C-Atomen, insbesondere für Methyl, oder eine Phenylgruppe, die mit einem oder mehreren Resten R³ substituiert sein kann.

Bevorzugte substituierte Ausführungsformen der Formel (28) sind die folgenden Formeln (28a) und (28b), bevorzugte Ausführungsformen der Formel (31) ist die Formel (31 a), bevorzugte Ausführungsformen der Formel (35) sind die folgenden Formeln (35a), (35b), (35c) und (35d), bevorzugte Ausführungsformen der Formel (36) sind die folgenden Formeln (36a), (36b), (36c) und (36d), bevorzugte Ausführungsformen der Formel (37) sind die folgenden Formeln (37a), (37b), (37c) und (37d), und bevorzugte Ausführungsformen der Formel (38) sind die folgenden Formeln (38a), (38b), (38c) und (38d), 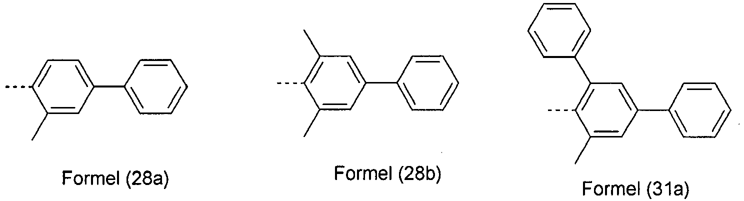 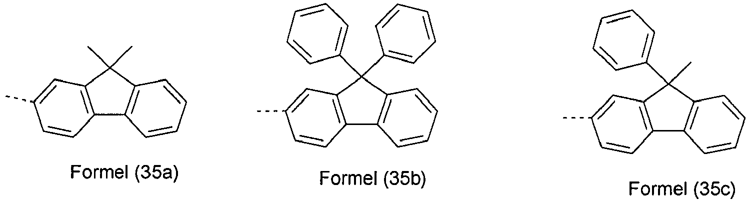 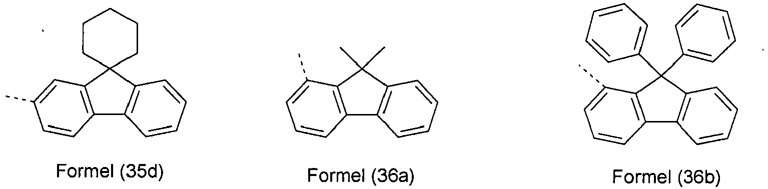 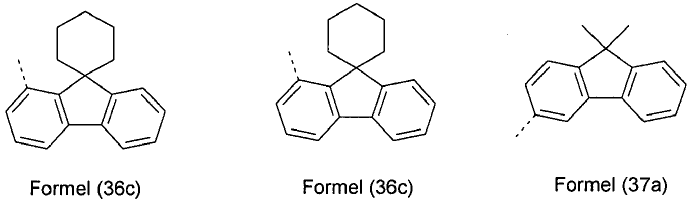 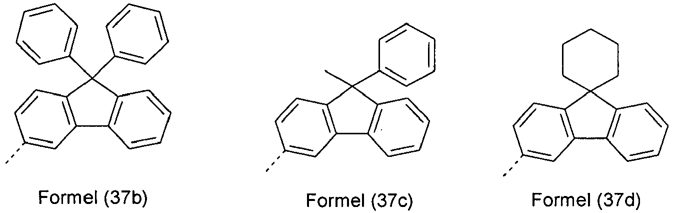 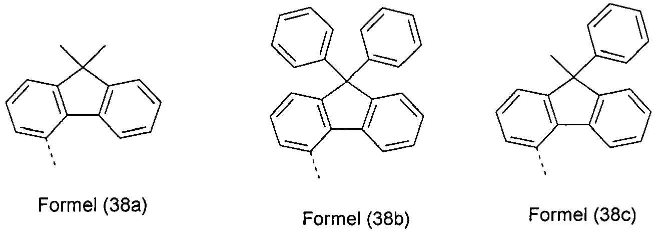 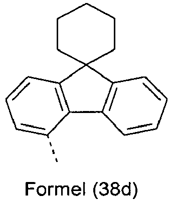 wobei die gestrichelte Bindung die Bindung an das Grundgerüst andeutet.

Wenn Y bzw. Y¹ bzw. Y² bzw. Y³ für C(R¹)₂ steht, so ist R¹ bevorzugt bei jedem Auftreten gleich oder verschieden ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, wobei eine oder mehrere nicht-benachbarte CH₂-Gruppen O oder S ersetzt sein können und wobei ein oder mehrere H-Atome durch D oder F ersetzt sein können, einem aromatischen Ringsystem mit 5 bis 24 aromatischen Ringatomen, das mit einem oder mehreren Resten R² substituiert sein kann, einem heteroaromatischen Ringsystem mit 5 bis 24 aromatischen Ringatomen, welches als Heteroarylgruppen ausschließlich schwefelhaltige oder sauerstoffhaltige Heteroarylgruppen enthält und welches mit einem oder mehreren Resten R² substituiert sein kann, oder einer Aralkyl- oder Heteroaralkylgruppe mit 5 bis 24 aromatischen Ringatomen, die mit einem oder mehreren Resten R² substituiert sein kann, wobei optional die Substituenten R¹, die an dasselbe Kohlenstoffatom gebunden sind, miteinander ein mono- oder polycyclisches, aliphatisches, aromatisches oder heteroaromatisches Ringsystem bilden und somit ein Spirosystem können, das mit einem oder mehreren Resten R² substituiert sein kann. Besonders bevorzugt ist R¹ bei jedem Auftreten gleich oder verschieden ausgewählt aus der Gruppe bestehend aus einer geradkettigen Alkylgruppe mit 1 bis 4 C-Atomen, insbesondere Methyl oder einer verzweigten Alkylgruppe mit 3 bis 10 C-Atomen, die jeweils mit einem oder mehreren Resten R² substituiert sein kann, oder einer Phenyl- oder ortho-, meta- oder para-Biphenylgruppe, die mit einem oder mehreren Resten R² substituiert sein kann, wobei optional die Substituenten R¹, die an dasselbe Kohlenstoffatom gebunden sind, miteinander ein mono- oder polycyclisches, aliphatisches, aromatisches oder heteroaromatisches Ringsystem und somit ein Spirosystem bilden können, das mit einem oder mehreren Resten R² substituiert sein kann.

Dabei haben für Verbindungen, die durch Vakuumverdampfung verarbeitet werden, die Alkylgruppen in den Resten R bzw. R¹ bevorzugt nicht mehr als vier C-Atome, besonders bevorzugt nicht mehr als ein C-Atom. Für Verbindungen, die aus Lösung verarbeitet werden, eignen sich insbesondere auch Verbindungen, die mit Alkylgruppen mit bis zu 10 C-Atomen substituiert sind oder die mit Oligoarylengruppen, beispielsweise ortho-, meta-, para- oder verzweigten Terphenylgruppen bzw. Quaterphenylgruppen oder ortho-, meta- oder para-Biphenylgruppen, substituiert sind.

In einer weiteren bevorzugten Ausführungsform der Erfindung ist L eine bivalente oder höher valente geradkettige Alkylen- oder Alkylidengruppe mit 1 bis 10 C-Atomen oder eine verzweigte oder cyclische Alkylen- oder Alkylidengruppe mit 3 bis 10 C-Atomen, die mit jeweils einem oder mehreren Resten R² substituiert sein kann, wobei ein oder mehrere H-Atome durch D oder F ersetzt sein können, oder ein mindestens bivalentes aromatisches Ringsystem mit 5 bis 24 aromatischen Ringatomen, welches durch einen oder mehrere Reste R² substituiert sein kann, oder ein oder heteroaromatisches Ringsystem mit 5 bis 24 aromatischen Ringatomen, welches als Heteroarylgruppen ausschließlich schwefelhaltige oder sauerstoffhaltige heteroaromatische Gruppen enthält und welches durch einen oder mehrere Reste R² substituiert sein kann, oder L ist eine chemische Bindung.

Die oben genannten Ausführungsformen der Erfindung sind beliebig miteinander kombinierbar. Insbesondere bevorzugt werden die oben als bevorzugt aufgeführten Ausführungsformen der Erfindung miteinander kombiniert.

Beispiele für bevorzugte Verbindungen gemäß den oben aufgeführten Ausführungsformen bzw. Verbindungen, wie sie bevorzugt in elektronischen Vorrichtungen eingesetzt werden können, sind die folgenden Verbindungen.

| | | |
|---|---|---|
| 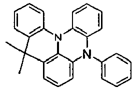 | 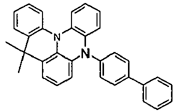 | 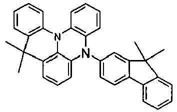 |
| 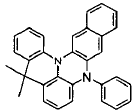 | 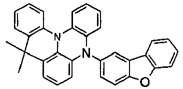 | 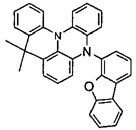 |
| 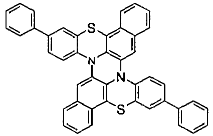 | 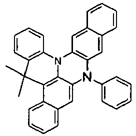 | 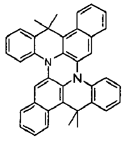 |
| 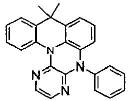 | 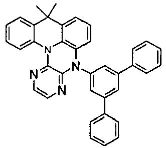 | 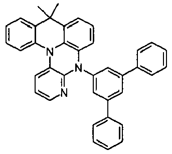 |
| 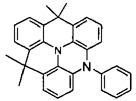 | 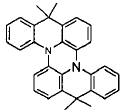 | 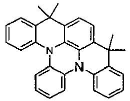 |
| 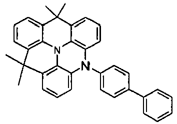 | 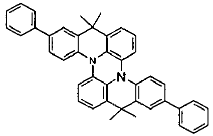 | 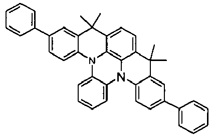 |
| 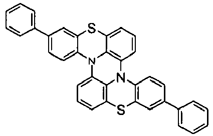 | 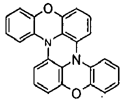 | 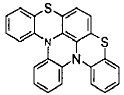 |
| 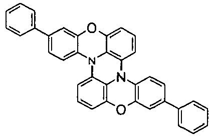 | 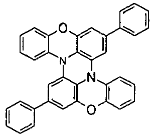 | 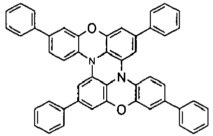 |
| 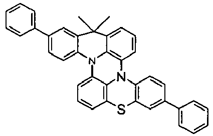 | 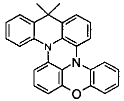 | 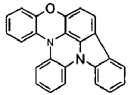 |
| 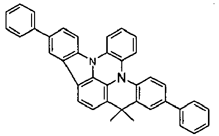 | 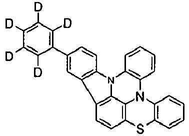 | 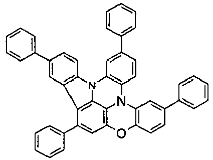 |
| 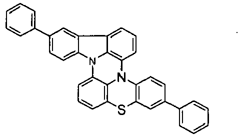 | 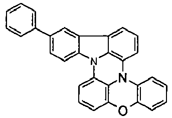 | 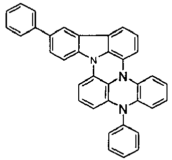 |
| | | *Nicht erfindungsgemäß |
| 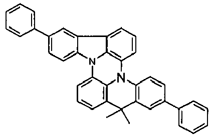 | 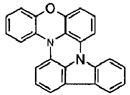 | 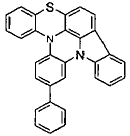 |
| 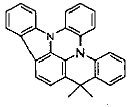 | 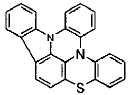 | 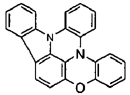 |
| 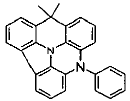 | 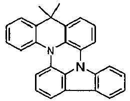 | 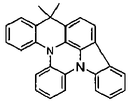 |
| 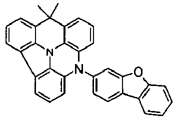 | 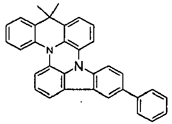 | 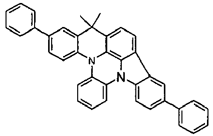 |
| 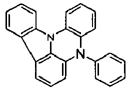 | 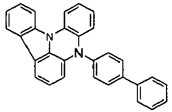 | 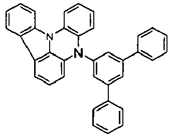 |
| 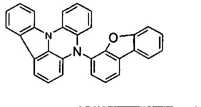 | 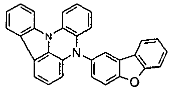 | 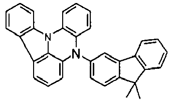 |
| 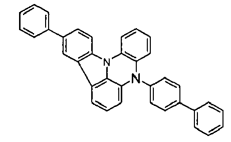 | 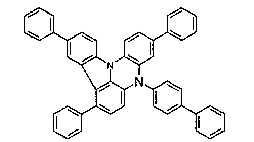 | 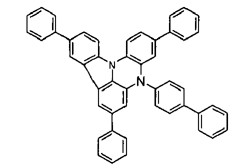 |
| 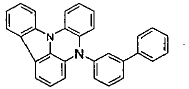 | 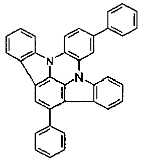 | 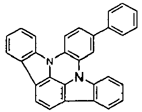 |
| 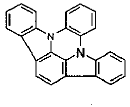 | 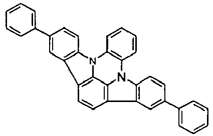 | 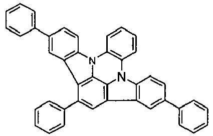 |
| 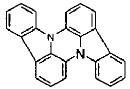 | 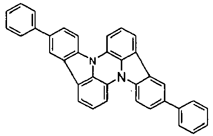 | 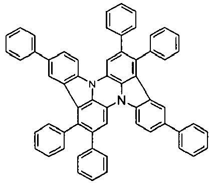 |
| 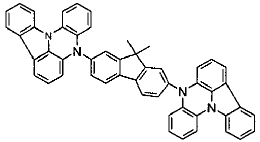 | 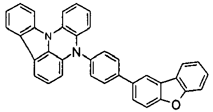 | 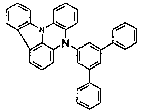 |
| 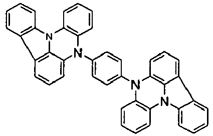 | 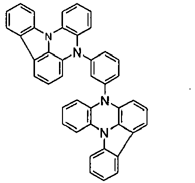 | 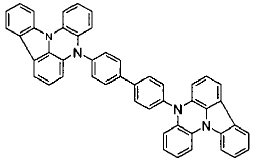 |
| 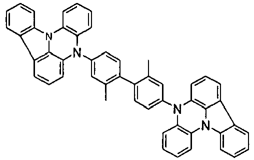 | 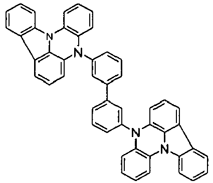 | 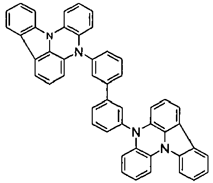 |
| 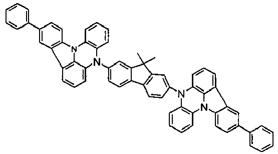 | 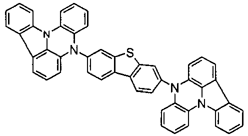 | 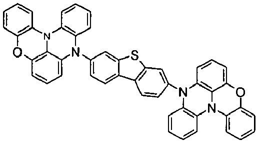 |
| 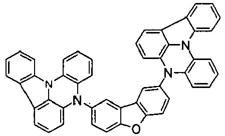 | 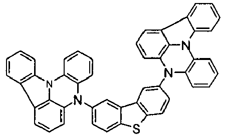 | 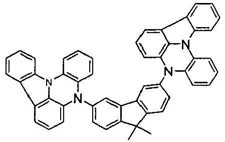 |
| 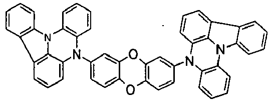 | 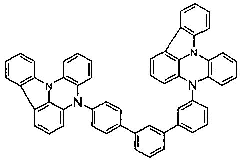 | 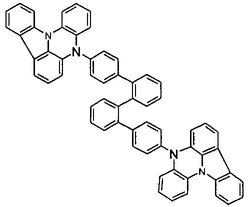 |
| 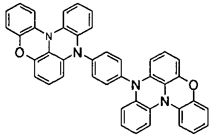 | 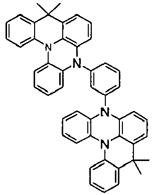 | 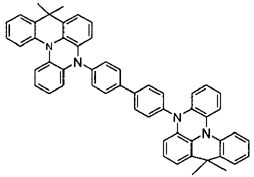 |
| 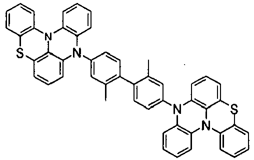 | 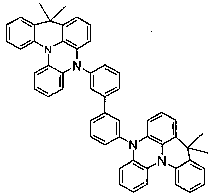 | 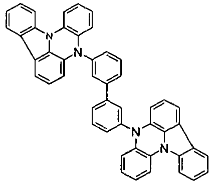 |
| 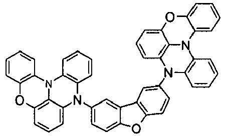 | 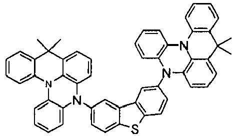 | 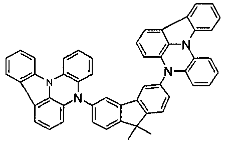 |
| 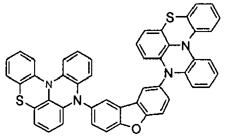 | 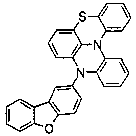 | 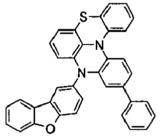 |
| 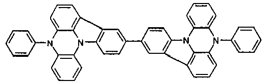 | 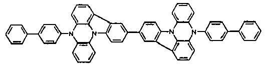 | 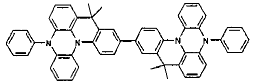 |
| 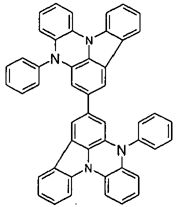 | 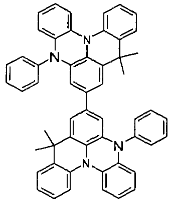 | 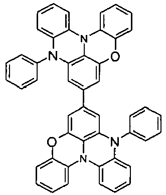 |
| 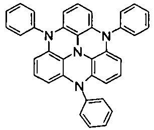 | 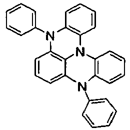 | 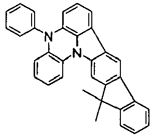 |
| *Nicht erfindungsgemäß | *Nicht erfindungsgemäß | |
| 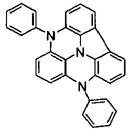 | | |
| *Nicht erfindungsgemäß | | |

Die Synthese der erfindungsgemäßen Verbindungen ist in Schema 1 bis Schema 3 allgemein gezeigt.

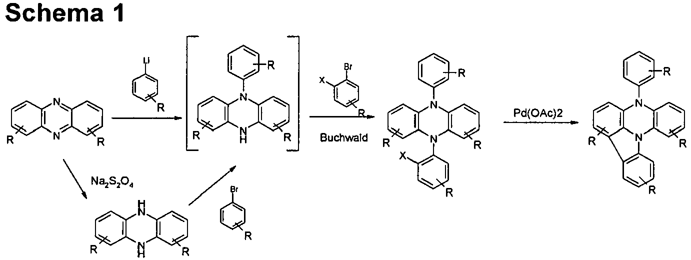

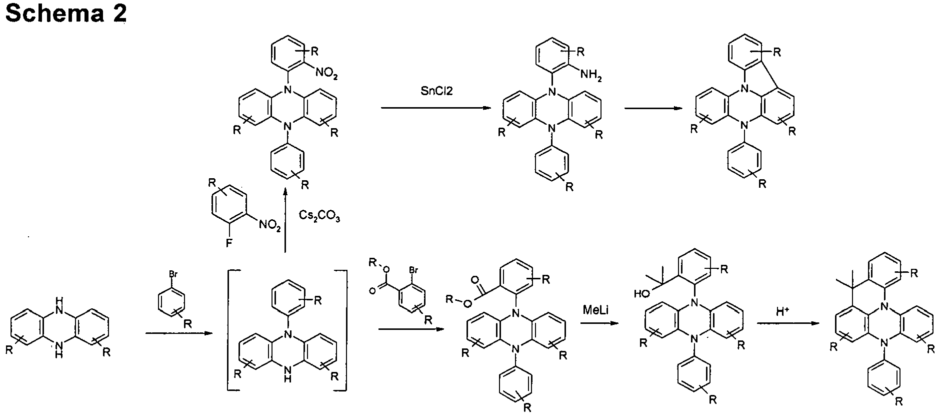

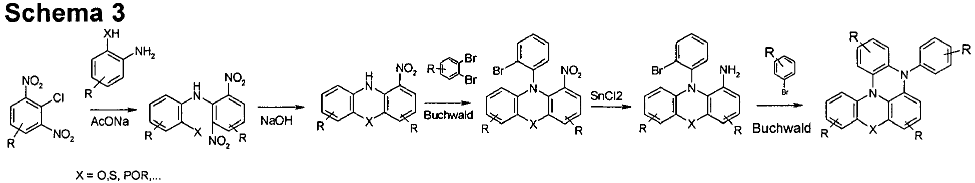

Ein weiterer Gegenstand der vorliegenden Erfindung ist daher ein Verfahren zur Herstellung einer Verbindung gemäß Formel (1), umfassend die Reaktionsschritte:
a) Synthese des entsprechenden Grundgerüsts, welche noch keine Brücke Y enthält; und
b) Einführung der Gruppe Y.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird. Geeignete fluoreszierende und phosphoreszierende Dotanden sind unten im Zusammenhang mit den organischen Elektrolumineszenzvorrichtungen aufgeführt und sind auch für die erfindungsgemäßen Mischungen bevorzugt. Die weitere Verbindung kann auch ein Dotierstoff sein, wenn die erfindungsgemäße Verbindung als Lochtransport- oder Elektronentransportverbindung eingesetzt wird. Geeignete Dotierstoffe sind unten im Zusammenhang mit den organischen Elektrolumineszenzvorrichtungen aufgeführt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der oben ausgeführten erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine erfindungsgemäse Verbindung als Matrixmaterial für fluoreszierende oder phosphoreszierende Emitter, insbesondere für phosphoreszierende Emitter, und/oder in einer elektronenblockierenden bzw. exzitonenblockierenden Schicht und/oder in einer Lochtransport- bzw. Lochinjektionsschicht. Dabei gelten die oben aufgeführten bevorzugten Ausführungsformen auch für die Verwendung der Materialien in organischen elektronischen Vorrichtungen.

In einer bevorzugten Ausführungsform der Erfindung wird die erfindungsgemäße Verbindung als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052 oder der nicht offen gelegten Anmeldung EP 11010103.7, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, oder überbrückte Carbazol-Derivate, z. B. gemäß US 2009/0136779, WO 2010/050778, WO 2011/042107 oder WO 2011/088877. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339 und WO 2012/007086 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die erfindungsgemäße Verbindung als Lochtransport- oder Lochinjektionsmaterial in einer Lochtransport- oder Lochinjektionsschicht eingesetzt. Dabei kann die emittierende Schicht fluoreszierend oder phosphoreszierend sein. Eine Lochinjektionsschicht im Sinne der vorliegenden Erfindung ist eine Schicht, die direkt an die Anode angrenzt. Eine Lochtransportschicht im Sinne der vorliegenden Erfindung ist eine Schicht, welche zwischen einer Lochinjektionsschicht und einer emittierenden Schicht vorliegt.

In nochmals einer weiteren bevorzugten Ausführungsform der Erfindung wird die erfindungsgemäße Verbindung in einer Exzitonenblockierschicht eingesetzt. Unter einer Exzitonenblockierschicht wird eine Schicht verstanden, die auf Anodenseite direkt an eine emittierende Schicht angrenzt.

Wenn die erfindungsgemäße Verbindung in einer Lochinjektions- bzw. in einer Lochtransportschicht eingesetzt werden, so können sie auch dotiert sein, wobei sich generell alle Dotanden eigenen, wie sie gemäß dem Stand der Technik üblicherweise eingesetzt werden. Geeignete Dotanden sind Elektronenakzeptor-Verbindungen, beispielsweise F₄-TCNQ (Tetrafluorotetracyano-chinodimethan) oder Verbindungen, wie sie in EP 1476881 oder EP 1596445 beschrieben werden.

In einer Ausführungsform der Erfindung wird die erfindungsgemäße Verbindung in einer Lochtransport- oder -injektionsschicht in Kombination mit einer Schicht, welche ein Hexaazatriphenylenderivat enthält, insbesondere Hexacyanohexaazatriphenylen (z. B. gemäß EP 1175470), verwendet. So ist beispielsweise eine Kombination bevorzugt, die folgendermaßen aussieht: Anode - Hexaazatriphenylenderivat - Lochtransportschicht, wobei die Lochtransportschicht eine oder mehrere erfindungsgemäße Verbindungen enthält. Ebenso ist es in diesem Aufbau möglich, mehrere aufeinander folgende Lochtransportschichten zu verwenden, wobei wenigstens eine Lochtransportschicht wenigstens eine erfindungsgemäße Verbindung enthält. Eine weitere bevorzugte Kombination sieht folgendermaßen aus: Anode - Lochtransportschicht - Hexaazatriphenylenderivat - Lochtransportschicht, wobei wenigstens eine der beiden Lochtransportschichten eine oder mehrere erfindungsgemäße Verbindungen enthält. Ebenso ist es in diesem Aufbau möglich, dass statt einer Lochtransportschicht mehrere aufeinander folgende Lochtransportschichten verwendet werden, wobei wenigstens eine Lochtransportschicht wenigstens eine erfindungsgemäße Verbindung enthält.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem / OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile aus:
1. Die erfindungsgemäßen Verbindungen, eingesetzt als Matrixmaterial für fluoreszierende oder phosphoreszierende Emitter führen zu hohen Effizienzen sowie zu langen Lebensdauern. Dies gilt insbesondere, wenn die Verbindungen als Matrixmaterial für einen phosphoreszierenden Emitter eingesetzt werden.
2. Die erfindungsgemäßen Verbindungen weisen eine hohe thermische Stabilität auf.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
4. Bei Verwendung als Lochtransportmaterial führen die erfindungsgemäßen Verbindungen zu guten Eigenschaften in Bezug auf die Effizienz, die Lebensdauer und die Betriebsspannung von organischen Elektrolumineszenzvorrichtungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Als Edukt kann z. B. Phenazin dienen. Die Angaben in eckigen Klammern bei den literaturbekannten Edukten beziehen sich auf die CAS-Nummer.

### Beispiel 1: 5,10-Dihydrophenazin

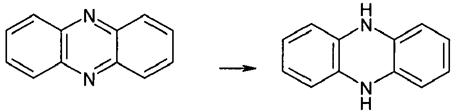

Unter Schutzgas werden 20 g (110 mmol) Phenazin in 600 ml Ethanol suspendiert. Die Reaktionsmischung wird unter Rückfluss erhitzt. Anschließend werden 38.3 g (220 mmol) Natriumdithionit gelöst in 600 ml entgastem Wasser zugetropft und weiter 4 h unter Rückfluss erhitzt. Nach Erkalten wird der ausgefallene gelbe Feststoff unter Schutzgas abfiltriert und in Vakuum getrocknet. Die Reinheit beträgt 92.0 %. Ausbeute: 19 g (107 mmol) 96 % der Theorie.

### Beispiel 2: 5-Biphenyl-4-yl-10-(2-bromphenyl)-5,10-dihydrophenazin

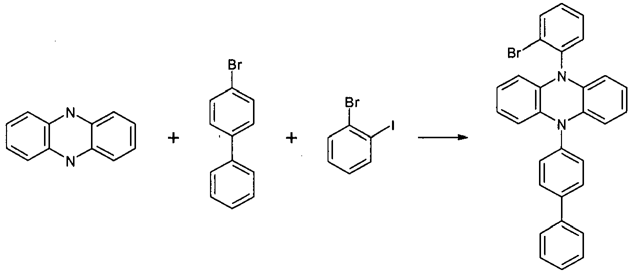

Unter Schutzgas werden 15.8 g (87.8 mmol) 9,10-Dihydrophenazin, 20 g (87 mmol) 4-Brombiphenyl und 0.8 g (0.88 mmol) Tris(dibenzyliden-aceton)dipalladium, 1.79 g (7.9 mmol) Palladiumacetat in 500 ml Toluol suspendiert. Die Reaktionsmischung wird 8 h unter Rückfluss erhitzt. Anschließend wird 24.8 g (87 mmol) 1-Brom-2-iod-benzol zugegeben und weitere 8 h unter Rückfluss erhitzt. Nach Erkalten wird die organische Phase abgetrennt, dreimal mit je 200 mL Wasser gewaschen und anschließend zur Trockene eingeengt. Das Produkt wird via Säulenchromatographie an Kieselgel mit Toluol/Heptan (1:2) gereinigt. Die Reinheit beträgt 97.0 %. Ausbeute: 29 g (37 mmol) 70 % der Theorie.

Analog werden die Verbindungen **2a - 2m** erhalten:

| **Bsp**. | **Edukt 1** | **Edukt 2** | **Produkt** | **Ausbeute** |
|---|---|---|---|---|
| 2a | 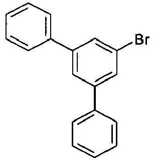 | 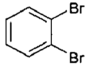 | 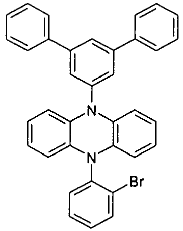 | 65% |
| | **[103068-20-8]** | | | |
| 2b | 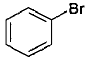 | 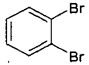 | 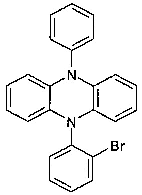 | 66% |
| 2c | 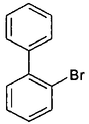 | 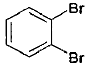 | 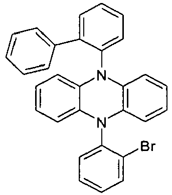 | 73% |
| | **[2052-07-5]** | | | |
| 2d | 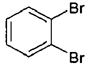 | 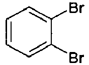 | 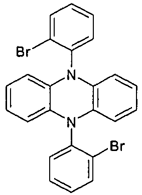 | |
| 2f | 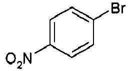 | 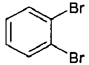 | 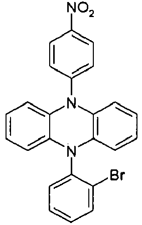 | 75% |
| | **[586-78-7]** | | | |
| 2g | 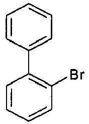 | 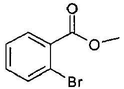 | 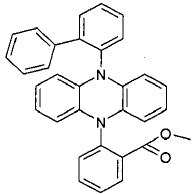 | 65% |
| | | **[610-94-6]** | | |
| 2h | 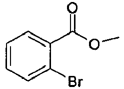 | 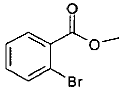 | 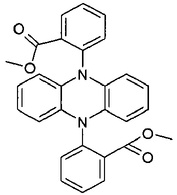 | 87% |
| 2i | 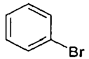 | 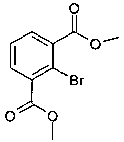 | 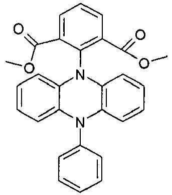 | 63% |
| 2j | 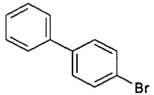 | 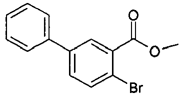 | 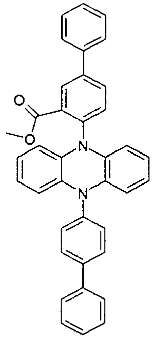 | 72% |
| | | [727408-92-6] | | |
| 2k | 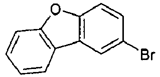 | 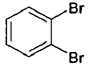 | 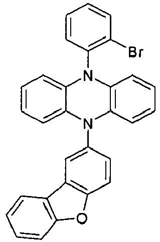 | 67% |
| | **[86-76-0**] | | | |
| 2l | 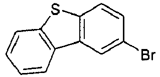 | 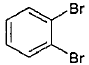 | | 69% |
| | [22439-61-8] | | 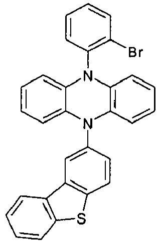 | |
| 2m | 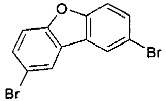 | 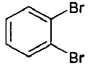 | 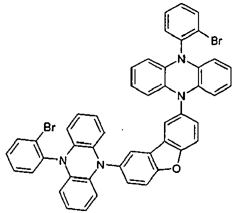 | 63% |
| | **[10016-52-1**] | | | |

Analog wird die Verbindung **2n** erhalten:

| **Bsp**. | **Edukt 1** | **Edukt 2** | **Edukt 3** | **Produkt** | **Ausbeute** |
|---|---|---|---|---|---|
| **2n** | 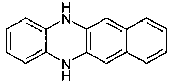 | 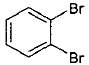 | 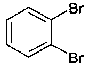 | 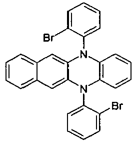 | **69%** |
| | [19029-32-4] | | | | |

### Beispiel 3: 8-Biphenyl-4-yl-8H-8,12b-diaza-benzo[a]aceanthrylen

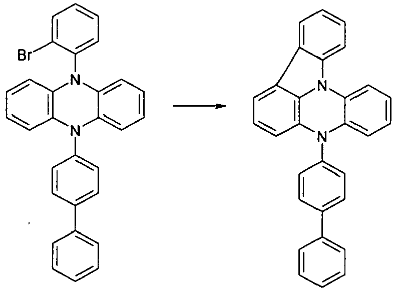

Unter Schutzgas werden 73 g (0.175 mmol) 5-Biphenyl-4-yl-10-(2-bromphenyl)-5,10-dihydrophenazin in 500 mL Dimethylacetamid gelöst. Zu dieser Lösung werden 2.4 g (6.5 mmol) Tricyclohexylphosphintetrafluoroborat und 701 mg (3.1 mmol) Pd(OAc)₂ zugegeben. Anschließend wird das Gemisch 9 h bei 120 °C gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird mit heißem Toluol extrahiert, aus Toluol umkristallisiert und abschließend im Hochvakuum sublimiert. Die Ausbeute beträgt 49 g (121 mmol), 81% der Theorie.

Analog werden die Verbindungen **3a** - **3m** erhalten:

| **Bsp**. | **Edukt 1** | **Produkt** | **Ausbeute** |
|---|---|---|---|
| 3a | 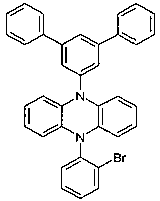 | 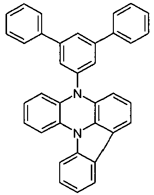 | 75% |
| 3b | 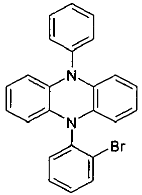 | 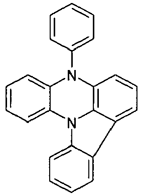 | 76% |
| 3c | 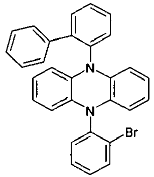 | 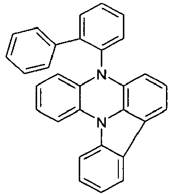 | 73% |
| 3e | 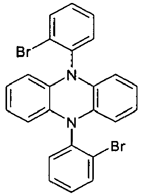 | 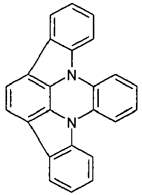 | 40% |
| 3g | 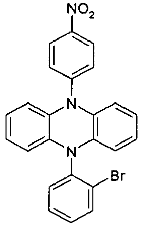 | 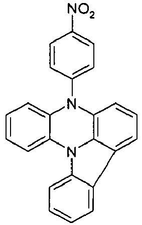 | 76% |
| 3h | 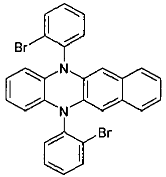 | 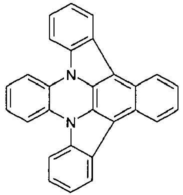 | 31% |
| 3i | 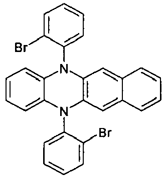 | 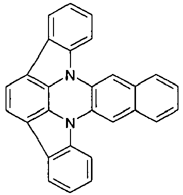 | 29% |
| 3j | 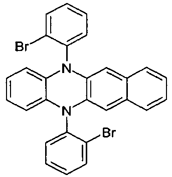 | 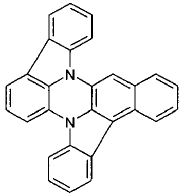 | 20% |
| 3k | 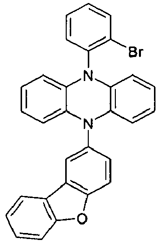 | 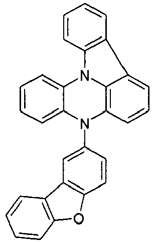 | 71% |
| 3l | 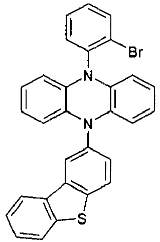 | 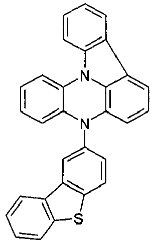 | 69% |
| 3m | 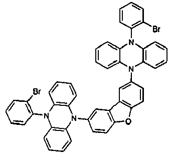 | 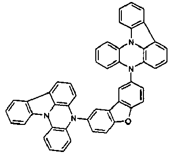 | 72% |

### Beispiel 4: 2-[2-(10-Biphenyl-2-yl-10H-phenazin-5-yl)-phenyl]-propan-2-ol

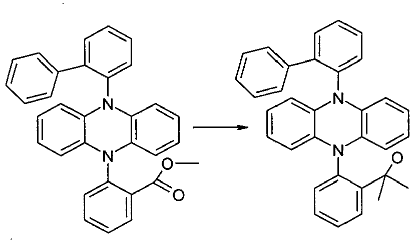

99 g (213 mmol) 2-(10-Biphenyl-2-yl-10H-phenazin-5-yl)-benzoesäure-methylester werden in 1500 mL getrocknetem THF gelöst und entgast. Es wird auf-78 °C gekühlt und innerhalb von 40 min. mit 569 mL (854 mmol) Methyllithium versetzt. Man lässt innerhalb 1 h bis auf -40 °C erwärmen und kontrolliert die Umsetzung via DC. Nach vollständiger Umsetzung wird bei -30 °C vorsichtig mit MeOH gequencht. Die Reaktionslösung wird auf 1/3 eingeengt und mit 1 L CH₂Cl₂ versetzt, gewaschen und die organische Phase über MgSO₄ getrocknet und eingeengt. Die Ausbeute beträgt 90 g (194 mmol) 90% der Theorie.

Analog werden die Verbindungen **4a** - **4c** erhalten

| **Bsp**. | **Edukt 1** | **Produkt** | **Ausbeute** |
|---|---|---|---|
| 4a | 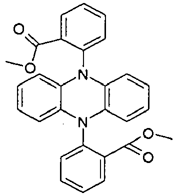 | 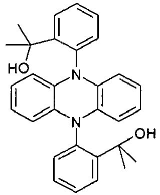 | 87% |
| 4b | 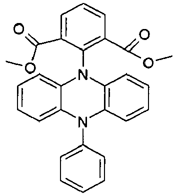 | 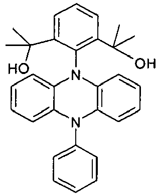 | 67% |
| 4c | 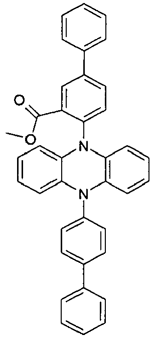 | 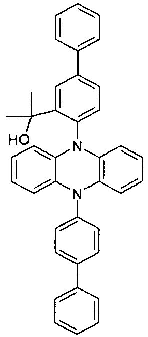 | 74% |

### Beispiel 5: 5-Biphenyl-2-yl-9,9-dimethyl-5H,9H-5,13b-diaza-naphtho [3,2,1-de]anthracen

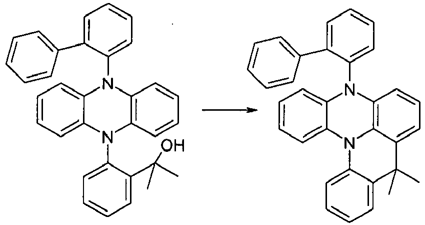

20 g (43.6 mmol) 2-[2-(10-Biphenyl-2-yl-10H-phenazin-5-yl)-phenyl]-propan-2-ol werden in 1200 mL entgastem Toluol gelöst und mit einer Suspension aus 40 g Polyphosphorsäure und 28 mL Methansulfonsäure versetzt und für 1 h auf 60 °C erhitzt. Der Ansatz wird abgekühlt und mit Wasser versetzt. Es fällt ein Feststoff aus, der in CH₂Cl₂/THF (1:1) gelöst wird. Die Lösung wird mit 20%iger NaOH vorsichtig alkalisiert, die Phasen werden getrennt und über MgSO₄ getrocknet. Der Rückstand wird mit Toluol heiß extrahiert, aus Toluol/Heptan (1:2) umkristallisiert und abschließend im Hochvakuum sublimiert. Die Ausbeute beträgt 15.6 g (34 mmol), 80 % der Theorie.

Analog werden die Verbindungen **5a - 5d** erhalten.

| **Bsp**. | **Edukt 1** | **Produkt** | **Ausbeute** |
|---|---|---|---|
| 5a | 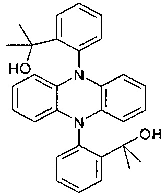 | 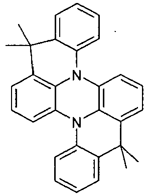 | 43% |
| 5b | 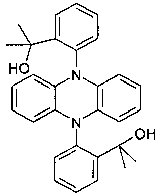 | 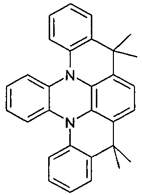 | 33% |
| 5c | 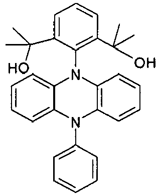 | 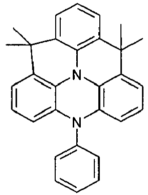 | 68% |
| 5d | 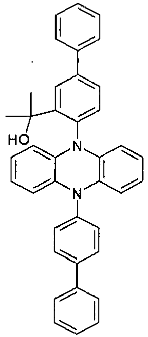 | 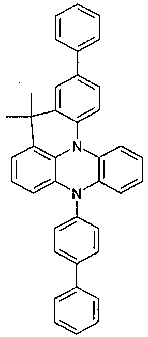 | 69% |

### Beispiel 6: 1-Nitro-10-(2-bromphenyl)-10H-phenoxazin

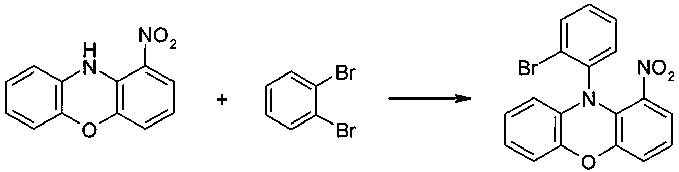

Unter Schutzgas werden 20 g (88 mmol) 1-Nitro-10H-phenoxazin (CAS: 26103-27-5), 20 g (88 mmol) 1,2-Dibrombenzol, 0.8 g (0.88 mmol) Tris-(dibenzylideneaceton)dipalladium und 1.79 g (7.9 mmol) Palladiumacetat in 500 ml Toluol suspendiert. Die Reaktionsmischung wird 8 h unter Rückfluss erhitzt. Nach Erkalten wird die organische Phase abgetrennt, dreimal mit 200 mL Wasser gewaschen und anschließend zur Trockene eingeengt. Das Produkt wird via Säulenchromatographie an Kieselgel mit Toluol/Heptan (1:2) gereinigt. Die Reinheit beträgt 97.0 %. Ausbeute: 19 g (49 mmol), 78 % der Theorie.

Analog werden die Verbindungen **6a-6b** erhalten:

| **Bsp**. | **Edukt** 1 | **Produkt** | **Ausbeute** |
|---|---|---|---|
| 6a | 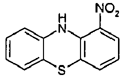 | 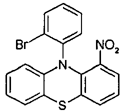 | 65% |
| | **[1747-87-1**] | | |
| 6b | 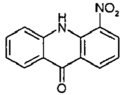 | 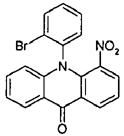 | 68% |
| | **[61-62-5** | | |

### Beispiel 7: 10-(2-Bromo-phenyl)-10H-phenoxazin-1-ylamine

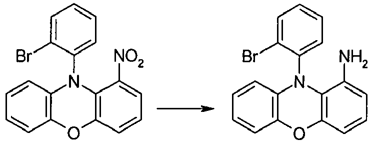

14.5 g (42 mmol) 1-Nitro-10-(2-bromphenyl)-10H-phenoxazin wird in 200 mL Ethanol suspendiert. Unter Rühren bei 60 °C werden 26 g (140 mmol) SnCl₂ gelöst in 25 ml konz. HCl portionsweise hinzugefügt und 8 h unter Rückfluss erhitzt. Danach wird der Niederschlag abfiltriert und im Vakuum getrocknet. Die Reinheit beträgt 94 %. Ausbeute: 12 g (35 mmol), 92 % der Theorie.

Analog werden die Verbindungen **7a-7b** erhalten:

| **Bsp**. | **Edukt 1** | **Produkt** | **Ausbeute** |
|---|---|---|---|
| 7a | 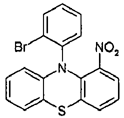 | 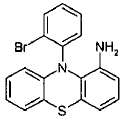 | 89% |
| 7b | 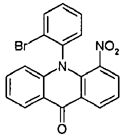 | 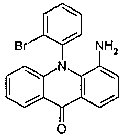 | 81% |

### Beispiel 8: 9-Phenyl-9H-5-oxa-9,13b-diaza-naphtho[3,2,1-de]anthracen

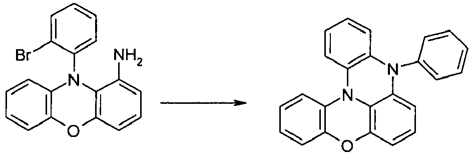

Unter Schutzgas werden 9.2 g (26.3 mmol) 9,10-Dihydrophenazin, 0.24 g (0.26 mmol) Tris(dibenzylideneaceton)dipalladium und 0.53 g (2.37 mmol) Palladiumacetat in 150 ml Toluol suspendiert. Die Reaktionsmischung wird 8 h unter Rückfluss erhitzt. Anschließend werden 4 g (26 mmol) 4-Brombenzol zugegeben und weitere 8 h unter Rückfluss erhitzt. Nach Erkalten wird die organische Phase abgetrennt, dreimal mit 80 mL Wasser gewaschen und anschließend zur Trockene eingeengt. Der Rückstand wird mit Toluol heiß extrahiert, aus Toluol/Heptan (1:2) umkristallisiert und abschließend im Hochvakuum sublimiert. Die Reinheit beträgt 97.0 %. Ausbeute: 5.6 g (16.3 mmol) 63 % der Theorie.

Analog werden die Verbindungen **8a-8f** erhalten:

| **Bsp**. | **Edukt 1** | **Edukt 2** | **Produkt** | **Ausbeute** |
|---|---|---|---|---|
| 8a | 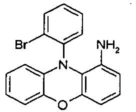 | 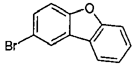 | 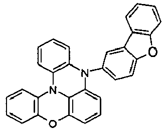 | 64% |
| 8b | 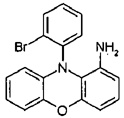 | 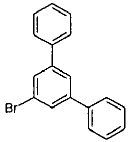 | 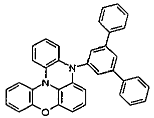 | 59% |
| | | **[103068-20-8]** | | |
| 8c | 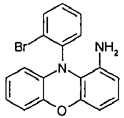 | 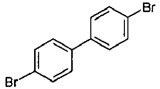 | 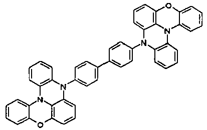 | 53% |
| 8e | 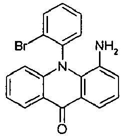 | 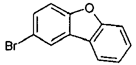 | 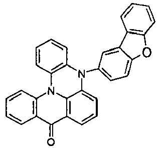 | 58% |
| 8f | 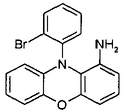 | 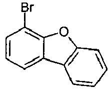 | 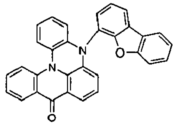 | 55% |
| | | **[89827-45-2]** | | |

### Beispiel 9: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E17 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind, werden zur verbesserten Prozessierung mit 20 nm PEDOT:PSS (Poly(3,4-ethylenedioxythiophen)poly(styrolsulfonat), bezogen als CLEVIOS™ P VP Al 4083 von Heraeus Precious Metals GmbH Deutschland, aus wässriger Lösung aufgeschleudert) beschichtet. Diese beschichteten Glasplättchen bilden die Substrate, auf welche die OLEDs aufgebracht werden. Die OLEDs haben prinzipiell folgenden Schichtaufbau: Substrat / optionale Lochinjektionsschicht (HIL) / Lochtransportschicht (HTL) / optionale Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 1 gezeigt. Eine Bezeichnung der Materialien wie "3f" bezieht sich weiterhin auf das Material, dessen Synthese im oben genannten Beispiel 3f beschrieben ist. Dies gilt analog für die anderen erfindungsgemäßen Verbindungen.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:3k:TEG1 (70%:25%:5%) bedeutet hierbei, dass das Material IC1 in einem Volumenanteil von 70%, das Material aus Beispiel 3k in einem Anteil von 25 Vol.-% und TEG1 in einem Anteil von 5 Vol.-% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiel V1-V3 sind Vergleichsbeispiele gemäß dem Stand der Technik, die Beispiele E1-E17 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen als Lochtransportmaterialien

Verwendet man die erfindungsgemäße Verbindung 3f statt der ähnlichen Verbindung H3 gemäß dem Stand der Technik als Lochtransportmaterial in einer OLED mit dem grünen Dotanden TEG1, so erhält man eine fast 20% bessere Leistungseffizienz (Beispiele V1 und E5).

### Verwendung von erfindungsgemäßen Verbindungen als Matrix-materialien in phosphoreszierenden OLEDs

Häufig erhält man durch die Verwendung von zwei Materialien als Matrix (Host) in OLEDs mit grün phosphoreszierenden Dotanden bessere Leistungsdaten als bei Einsatz eines Einzelmaterials, weshalb Materialien für diesen Einsatzzweck von großem Interesse sind. Hierbei zeichnen sich u.a. erfindungsgemäße Materialien mit schwefel- bzw. sauerstoffhaltigen heteroaromatischen Ringsystemen als Substituent gegenüber triazinhaltigen Materialien gemäß dem Stand der Technik aus: Man erhält eine bessere Quanten- und Leistungseffizienz (Beispiele V2, V3, E6, E7 und E8). Weiterhin ist die Lebensdauer deutlich verbessert. Während bei Betrieb mit konstanter Stromdichte die Leuchtdichte für die Beispiele V2 und V3 innerhalb von 160 h bzw. 180 h von ihrem Startwert von 10000 cd/m² auf 70% abfällt, dauert dies in den Beispielen E6, E7 und E8 300 h, 245 h bzw. 230 h.

**Tabelle 1: Aufbau der OLEDs**

| Bsp. | HIL Dicke | HTL Dicke | IL Dicke | EBL Dicke | EML Dicke | HBL Dicke | ETL Dicke | EIL Dicke |
|---|---|---|---|---|---|---|---|---|
| V1 | --- | SpA1 70nm | HATCN 5nm | H3 90nm | IC1:TEG1 (90%:10%) 30nm | --- | ST1:LiQ (50%:50%) 40nm | --- |
| V2 | --- | SpA1 70nm | HATCN 5nm | SpMA1 90nm | IC1:H1:TEG1 (70%:25%:5%) 30nm | IC1 10nm | ST1:LiQ (50%:50%) 30nm | --- |
| V3 | --- | SpA1 70nm | HATCN 5nm | SpMA1 90nm | IC1:H2:TEG1 (70%:25%:5%) 30nm | IC1 10nm | ST1:LiQ (50%:50%) 30nm | --- |
| E1 | --- | SpA1 90nm | HATCN 5nm | SpMA1 130nm | IC5:3c:TER1 (43%:50%:7%) 40nm | --- | ST2:LiQ (50%:50%) 40nm | --- |
| E2 | --- | SpA1 90nm | HATCN 5nm | SpMA1 130nm | IC5:3i:TER1 (33%:60%:7%) 40nm | --- | ST2:LiQ (50%:50%) 40nm | --- |
| E3 | --- | SpA1 90nm | HATCN 5nm | SpMA1 130nm | IC5:5:TER1 (43%:50%:7%) 40nm | --- | ST2:LiQ (50%:50%) 40nm | --- |
| E4 | --- | SpA1 90nm | HATCN 5nm | SpMA1 130nm | IC5:5d:TER1 (53%:40%:7%) 40nm | IC1 10nm | ST2:LiQ (50%:50%) 30nm | --- |
| E5 | --- | SpA1 70nm | HATCN 5nm | 3m 90nm | IC1:TEG1 (90%:10%) 30nm | --- | ST1:LiQ (50%:50%) 40nm | --- |
| E6 | --- | SpA1 70nm | HATCN 5nm | SpMA1 90nm | IC1:3k:TEG1 (70%:25%:5%) 30nm | IC1 10nm | ST1:LiQ (50%:50%) 30nm | --- |
| E7 | --- | SpA1 70nm | HATCN 5nm | SpMA1 90nm | IC1:31:TEG1 (70%:25%:5%) 30nm | IC1 10nm | ST1:LiQ (50%:50%) 30nm | --- |
| E8 | --- | SpA1 70nm | HATCN 5nm | SpMA1 90nm | IC1:3m:TEG1 (70%:25%:5%) 30nm | IC1 10nm | ST1:LiQ (50%:50%) 30nm | --- |
| E9 | HATCN 5nm | 3a 140nm | HATCN 5nm | SpMA1 20nm | M2:D1 (95%:5%) 20nm | --- | ST1:LiQ (50%:50%) 30nm | LiQ 1nm |
| E10 | HATCN 5nm | 5a 140nm | HATCN 5nm | SpMA1 20nm | M2:D1 (95%:5%) 20nm | --- | ST1:LiQ (50%:50%) 30nm | LiQ 1nm |
| E11 | --- | 5c 70nm | HATCN 5nm | SpMA1 90nm | IC1:TEG1 (90%:10%) 30nm | --- | ST1:LiQ (50%:50%) 40nm | --- |
| E12 | HATCN 5nm | 8a 140nm | HATCN 5nm | SpMA1 20nm | M2:D1 (95%:5%) 20nm | --- | ST1:LiQ (50%:50%) 30nm | LiQ 1nm |
| E13 | HATCN 5nm | 8b 140nm | HATCN 5nm | SpMA1 20nm | M2:D1 (95%:5%) 20nm | --- | ST1:LiQ (50%:50%) 30nm | LiQ 1nm |
| E14 | HATCN 5nm | 8c 140nm | HATCN 5nm | SpMA1 20nm | M2:D1 (95%:5%) 20nm | --- | ST1:LiQ (50%:50%) 30nm | LiQ 1nm |
| E15 | HATCN 5nm | 8d 140nm | HATCN 5nm | SpMA1 20nm | M2:D1 (95%:5%) 20nm | --- | ST1:LiQ (50%:50%) 30nm | LiQ 1nm |
| E16 | --- | 8b 70nm | HATCN 5nm | SpMA1 90nm | IC1:TEG1 (90%:10%) 30nm | --- | ST1:LiQ (50%:50%) 40nm | --- |

**Tabelle 2: Daten der OLEDs**

| Bsp. | U1000 (V) | SE1000 (cd/A) | LE1000 (Im/W) | EQE 1000 | CIE x/y bei 1000 cd/m² |
|---|---|---|---|---|---|
| V1 | 3.7 | 55 | 47 | 15.3% | 0.33/0.62 |
| V2 | 3.7 | 53 | 45 | 14.8% | 0.33/0.62 |
| V3 | 3.8 | 45 | 37 | 12.7% | 0.32/0.61 |
| E1 | 4.7 | 10.8 | 7.2 | 11.7% | 0.67/0.33 |
| E2 | 5.1 | 11.7 | 7.2 | 12.6% | 0.67/0.33 |
| E3 | 4.9 | 10.4 | 6.7 | 11.3% | 0.67/0.33 |
| E4 | 4.7 | 10.0 | 6.7 | 10.8% | 0.67/0.33 |
| E5 | 3.4 | 59 | 55 | 16.5% | 0.33/0.62 |
| E6 | 3.9 | 57 | 46 | 15.8% | 0.33/0.62 |
| E7 | 3.5 | 60 | 53 | 16.7% | 0.33/0.62 |
| E8 | 3.6 | 56 | 49 | 16.0% | 0.31/0.61 |
| E9 | 4.3 | 9.5 | 6.9 | 7.4% | 0.14/0.15 |
| E10 | 4.4 | 10.2 | 7.2 | 7.8% | 0.14/0.16 |
| E11 | 3.6 | 57 | 51 | 16.1% | 0.32/0.62 |
| E12 | 4.5 | 10.1 | 7.1 | 7.8% | 0.14/0.16 |
| E13 | 4.1 | 9.1 | 6.9 | 7.0% | 0.14/0.16 |
| E14 | 4.2 | 10.5 | 7.8 | 8.1% | 0.14/0.16 |
| E15 | 4.3 | 9.5 | 7.0 | 7.3% | 0.14/0.16 |
| E16 | 3.5 | 58 | 52 | 15.8% | 0.33/0.62 |

**Tabelle 3: Strukturformeln der Materialien für die OLEDs**

| | |
|---|---|
| 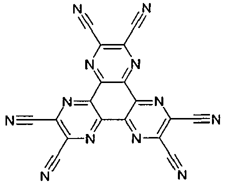 | 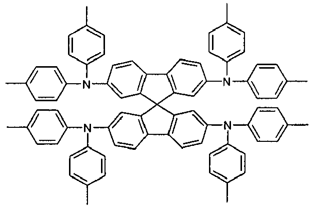 |
| HATCN | SpA1 |
| 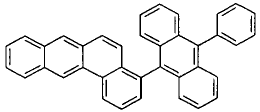 | 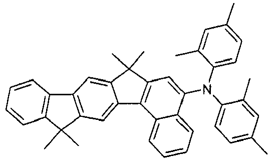 |
| M2 | D1 |
| 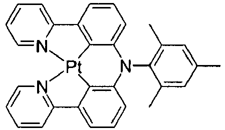 | 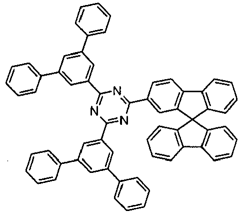 |
| TER1 | ST1 |
| 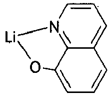 | 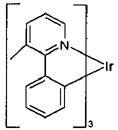 |
| LiQ | TEG1 |
| 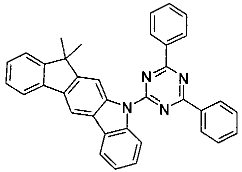 | 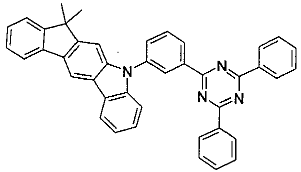 |
| IC1 | IC5 |
| 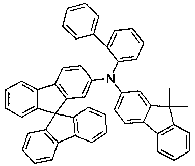 | 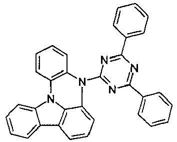 |
| SpMA1 | H1 (Stand der Technik) |
| 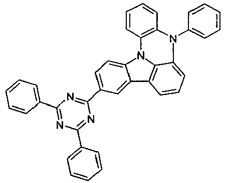 | 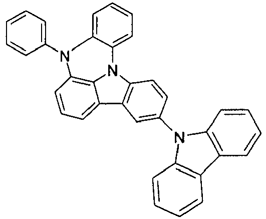 |
| H2 (Stand der Technik) | H3 (Stand der Technik) |

## Patentansprüche

1. Verbindung gemäß Formel (1 a) oder (2a), 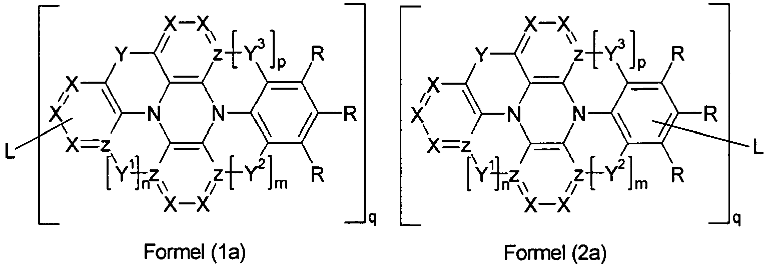 wobei für die verwendeten Symbole und Indizes gilt:
X ist bei jedem Auftreten gleich oder verschieden CR oder N; bzw. X steht für C, wenn an diese Gruppe X eine Gruppe L gebunden ist;
Y, Y¹, Y², Y³ ist bei jedem Auftreten gleich oder verschieden eine Einfachbindung oder C(R¹)₂, O, oder S; mit der Maßgabe, dass Y und Y¹ nicht gleichzeitig für eine Einfachbindung stehen und dass Y² und Y³ nicht gleichzeitig für eine Einfachbindung stehen;
Z ist bei jedem Auftreten gleich oder verschieden CR oder N; bzw. Z steht für C, wenn an diese Gruppe Z eine Gruppe Y¹ bzw. Y² bzw. Y³ gebunden ist;
L ist gleich oder verschieden R, wenn q = 1 ist, oder ist eine bi-, tri-, tetra-, penta- oder hexavalente geradkettige Alkylen-, Alkyliden-, Alkylenoxy- oder Thioalkylenoxygruppe mit 1 bis 40 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R² substituiert sein kann, wobei jeweils eine oder mehrere nicht benachbarte CH₂-Gruppen durch -R²C=CR²-, -C=C-, Si(R²)₂, C=O, C=NR², P(=O)R², S=O, SO₂, -O-, -S- oder -CONR²- ersetzt sein können und wobei ein oder mehrere H-Atome durch D, F, Cl, Br, I, CN oder NO₂ ersetzt sein können, oder ein bi-, tri-, tetra-, penta- oder hexavalentes aromatisches Ringsystem mit 5 bis 40, aromatischen Ringatomen, welches durch einen oder mehrere Reste R² substituiert sein kann, oder heteroaromatisches Ringsystem mit 5 bis 60 aromatischen Ringatomen, welches als Heteroarylgruppen ausschließlich schwefelhaltige oder sauerstoffhaltige Heteroarylgruppen enthält und welches durch einen oder mehrere Reste R² substituiert sein kann, oder P(R²)₃₋ᵣ, P(=O)(R²)₃₋ᵣ, C(R²)₄₋ᵣ, Si(R²)₄₋ᵣ, N(Ar)₃₋ᵣ, wobei r für 2, 3 oder 4 steht, mit der Maßgabe, dass r nicht größer ist als die maximale Valenz von L; oder L ist eine chemische Bindung, wobei dann q = 2 ist; dabei ist die Valenz der Gruppe L = q + 1;
n, m, p ist bei jedem Auftreten gleich oder verschieden 0 oder 1, wobei n = 0 bzw. m = 0 bzw. p = 0 bedeutet, dass die entsprechende Gruppe Y¹ bzw. Y² bzw. Y³ nicht vorhanden ist und dass statt Y² bzw. Y³ an dem entsprechenden Kohlenstoffatom eine Gruppe R gebunden ist;
q ist 1, 2, 3, 4, 5 oder 6;
R, R¹ ist bei jedem Auftreten gleich oder verschieden ausgewählt aus der Gruppe bestehend aus H, D, F, Cl, Br, I, CN, NO₂, C(=O)Ar, C(=O)R², P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen Ringsystem mit 6 bis 60 aromatischen Ringatomen, das jeweils mit einem oder mehreren Resten R² substituiert sein kann, einem heteroaromatischen Ringsystem mit 5 bis 60 aromatischen Ringatomen, welches als Heteroarylgruppen schwefelhaltige oder sauerstoffhaltige Heteroarylgruppen enthält und welches mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Aralkyl- oder Heteroaralkylgruppe mit 5 bis 60 aromatischen Ringatomen, die mit einem oder mehreren Resten R² substituiert sein kann, wobei optional zwei Substituenten R¹, die in derselben Gruppe Y gebunden sind, miteinander ein mono- oder polycyclisches, aliphatisches, aromatisches oder heteroaromatisches Ringsystem bilden können, das mit einem oder mehreren Resten R² substituiert sein kann; weiterhin können zwei benachbarte Reste R einen ankondensierten Benzoring bilden, der mit einem oder mehreren Resten R² substituiert sein kann; dabei ist die Gruppe R in Formel (2) nicht vorhanden, wenn an das entsprechende Kohlenstoffatom die Gruppe L bindet;
R² ist bei jedem Auftreten gleich oder verschieden ausgewählt aus der Gruppe bestehend aus H, D, F, Cl, Br, I, CN, NO₂, C(=O)Ar, C(=O)R³, P(=O)(Ar)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder eine Aralkyl- oder Heteroaralkylgruppe mit 5 bis 60 aromatischen Ringatomen;
Ar ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar, welche an dasselbe P-Atom binden, auch durch eine Einfachbindung oder eine Brücke, ausgewählt aus N(R³), C(R³)₂, O oder S, miteinander verbrückt sein;
R³ ist ausgewählt aus der Gruppe bestehend aus H, D, F, CN, einem aliphatischem Kohlenwasserstoffrest mit 1 bis 20 C-Atomen oder einem aromatischem Ringsystem mit 5 bis 30 aromatischen Ringatomen, in dem ein oder mehrere H-Atome durch D, F, Cl, Br, I oder CN ersetzt sein können;
dabei sind die folgenden Verbindungen von der Erfindung ausgenommen:

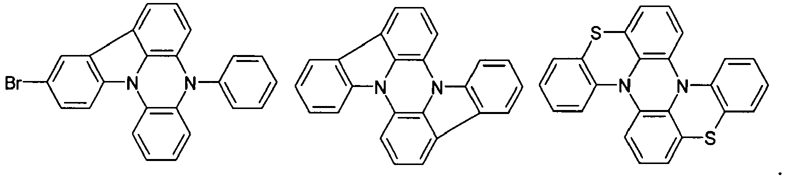

2. Verbindung nach Anspruch 1 oder 2, **dadurch gekennzeichnet, dass**
X, Z ist CR oder N, mit der Maßgabe, dass maximal eine der Gruppen X und Z pro Cyclus für N und die anderen Gruppen X und Z in diesem Cyclus stehen für CR, bzw. Z steht für C, wenn an dieses Z eine Gruppe Y¹ bzw. Y² bzw. Y³ gebunden ist, und X steht für C, wenn an dieses X eine Gruppe L gebunden ist.

3. Verbindung nach einem oder mehreren der Ansprüche 1 bis 3, **dadurch gekennzeichnet, dass** die Verbindung ausgewählt ist aus den Verbindungen der Formeln (1 b) oder (2b), 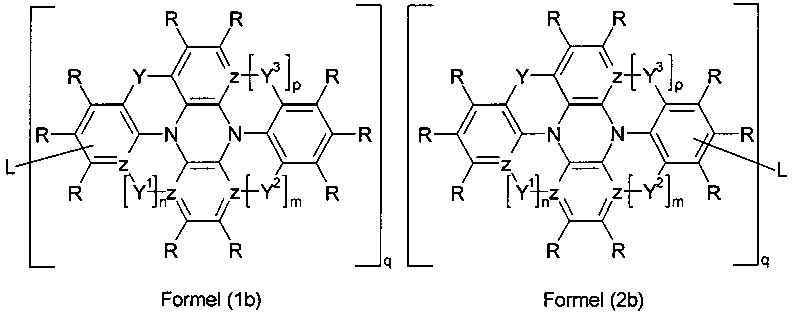 wobei für die verwendeten Symbole gilt:
Y, Y¹, Y², Y³ ist gleich oder verschieden bei jedem Auftreten eine Einfachbindung, C(R¹)₂, O oder S, insbesondere eine Einfachbindung oder C(R¹)₂;
Z ist CR, bzw. Z ist C, wenn an dieses Z eine Gruppe Y¹ bzw. Y² bzw. Y³ gebunden ist;
und die weiteren verwendeten Symbole und Indizes die in Anspruch 1 genannten Bedeutungen aufweisen; dabei ist der Rest R nicht vorhanden, wenn in dieser Position eine Gruppe L gebunden ist.

4. Verbindung nach einem oder mehreren der Ansprüche 1 bis 4, **dadurch gekennzeichnet, dass** die Verbindung ausgewählt ist aus den Verbindungen der Formeln (3) bis (8), 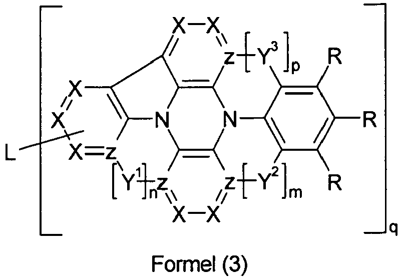 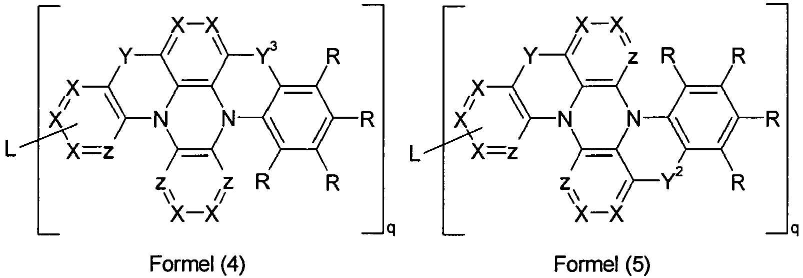 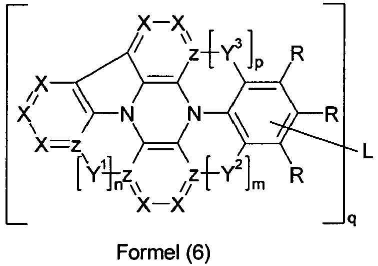 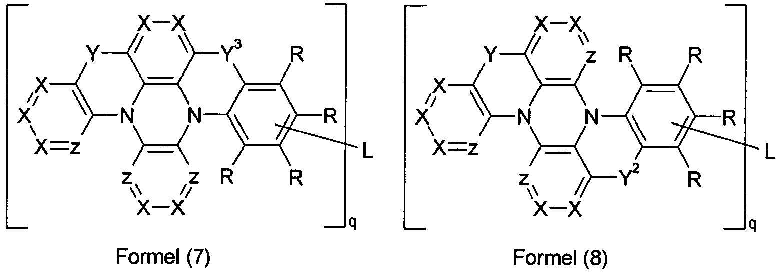 wobei die verwendeten Symbole und Indizes die in Anspruch 1 genannten Bedeutungen aufweisen und die Gruppe R nicht vorhanden ist, wenn in dieser Position eine Gruppe L gebunden ist.

5. Verbindung nach einem oder mehreren der Ansprüche 1 bis 5, **dadurch gekennzeichnet, dass** die Verbindung ausgewählt ist aus den Verbindungen gemäß Formel (3a), (4a) oder (5a), 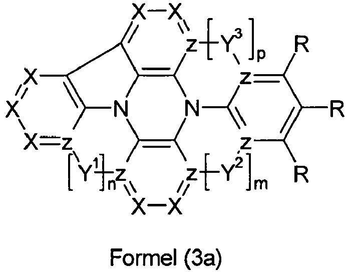 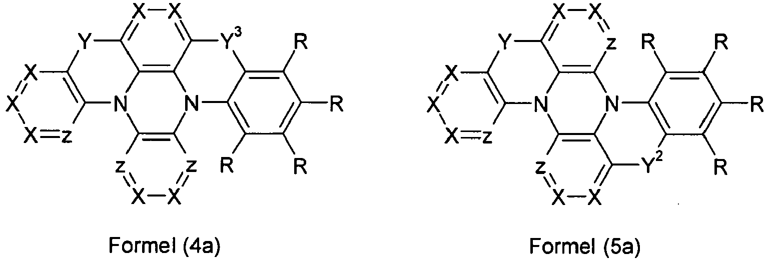 wobei die verwendeten Symbole und Indizes die in Anspruch 1 genannten Bedeutungen aufweisen.

6. Verbindung nach einem oder mehreren der Ansprüche 1 bis 6, **dadurch gekennzeichnet, dass** die Verbindung der Formel (3) ausgewählt ist aus den Verbindungen der (19) bis (23), 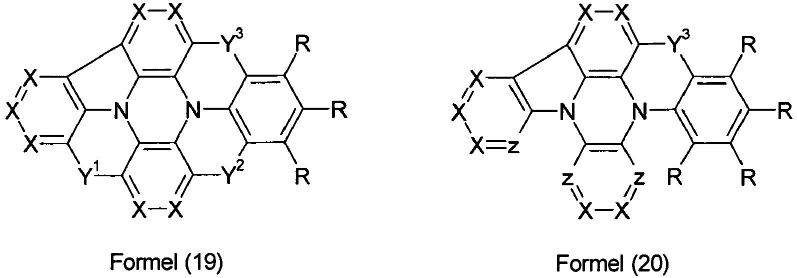 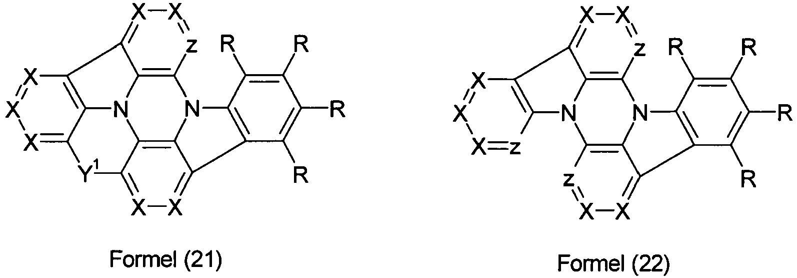 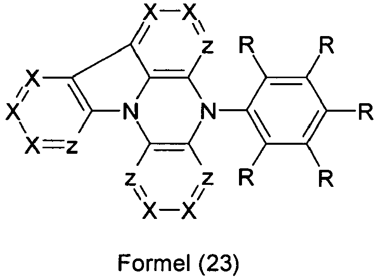 wobei die verwendeten Symbole und Indizes die in Anspruch 1 genannten Bedeutungen aufweisen.

7. Verbindungen nach einem oder mehreren der Ansprüche 1 bis 7, **dadurch gekennzeichnet, dass** die Verbindungen ausgewählt sind aus den Verbindungen der Formeln (19a) bis (23a), (24) oder (25), 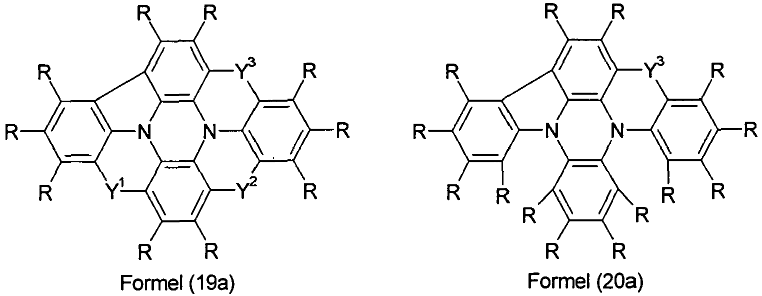 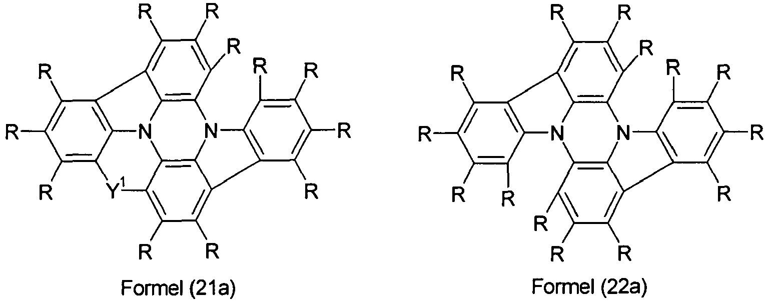 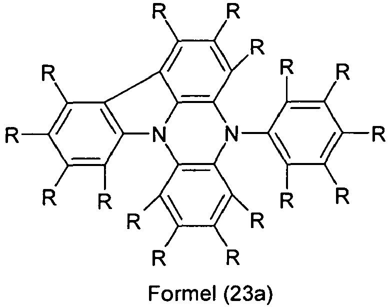 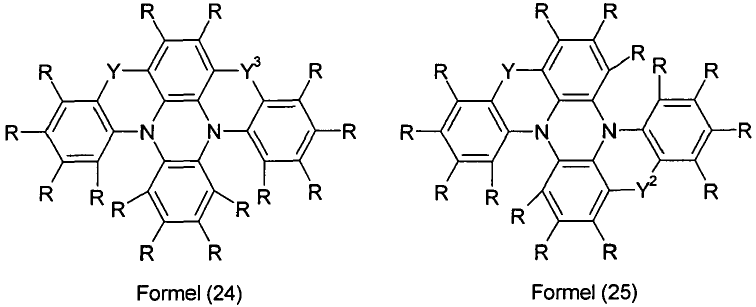 wobei die verwendeten Symbole die unter Anspruch 1 genannten Bedeutungen aufweisen.

8. Verbindung nach einem oder mehreren der Ansprüche 1 bis 8, **dadurch gekennzeichnet, dass** die Verbindung ausgewählt ist aus den Verbindungen der Formeln (19b) bis (23b), (24a) oder (25a), 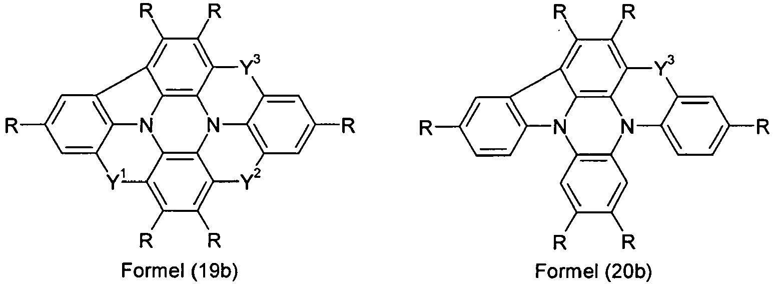 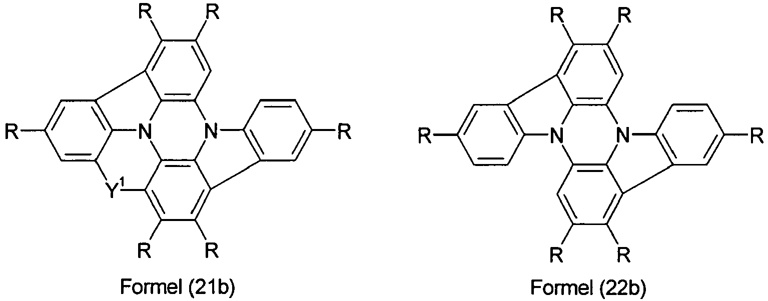 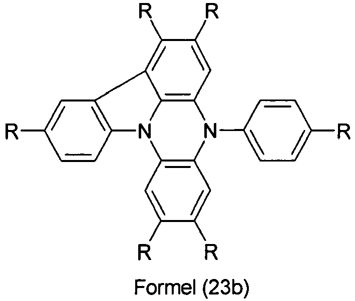 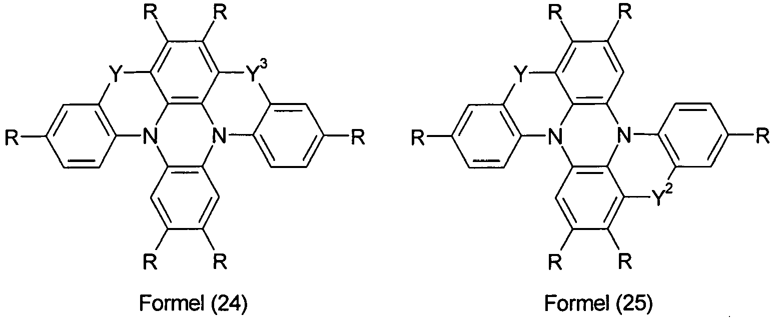 wobei die verwendeten Symbole die in Anspruch 1 genannten Bedeutungen aufweisen.

9. Verbindung nach einem oder mehreren der Ansprüche 1 bis 10, **dadurch gekennzeichnet, dass** R, wenn R für ein aromatisches oder heteroaromatisches Ringsystem steht, ausgewählt ist aus den Gruppen der Formeln (26) bis (58), 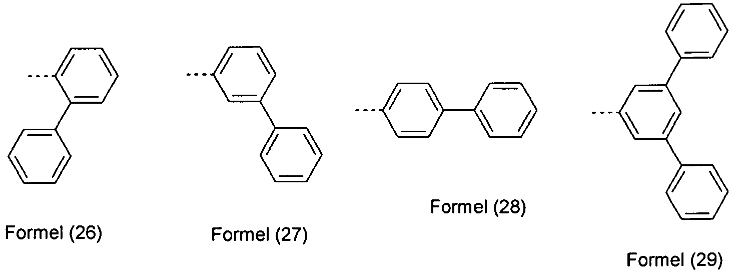 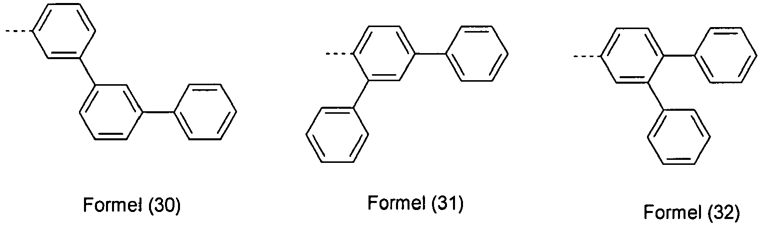 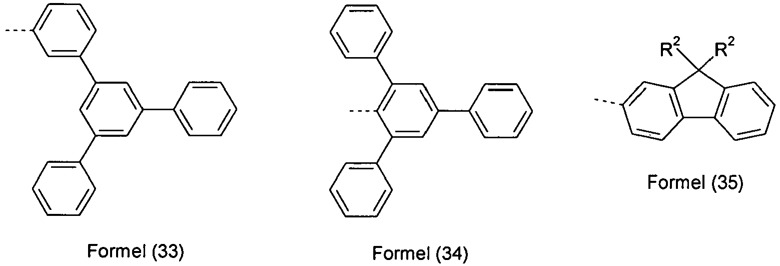 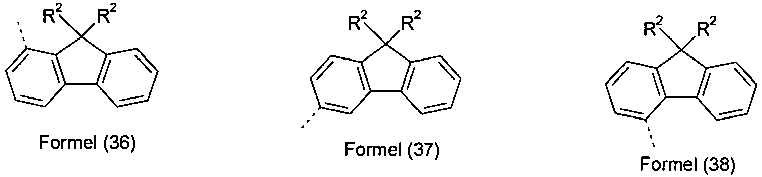 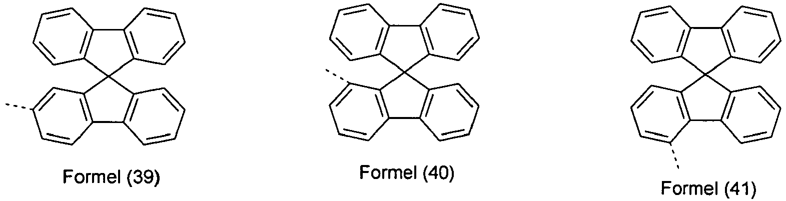 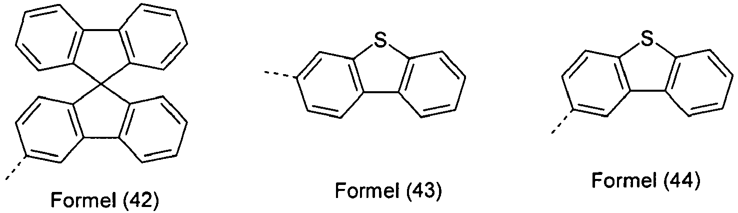 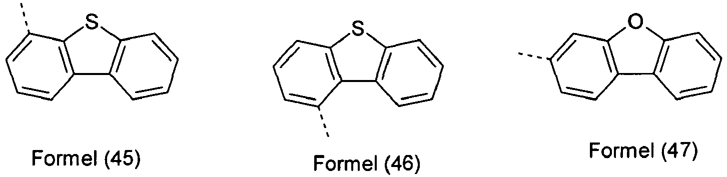 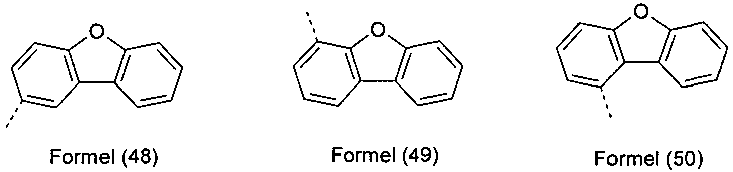 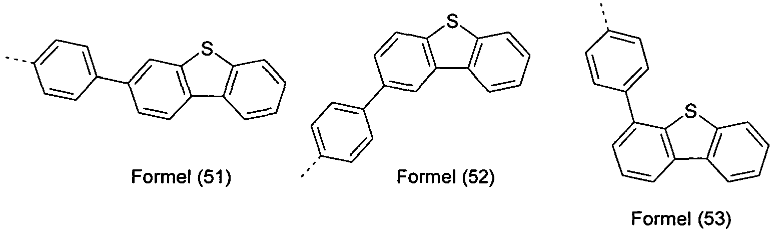 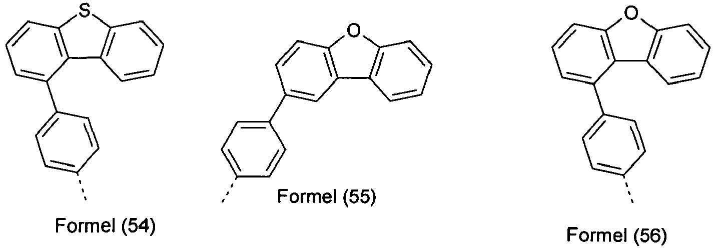 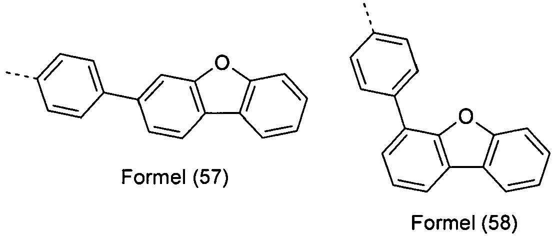 wobei die gestrichelte Bindung die Bindung an das Grundgerüst andeutet und die Gruppen durch einen oder mehrere Reste R² substituiert sein können.

10. Verfahren zur Herstellung einer Verbindung nach einem oder mehreren der Ansprüche 1 bis 10, umfassend die Reaktionsschritte:
a) Synthese einer Verbindung der Formel (1a) oder (1b), welche noch keine Brücke Y enthält; und
b) Einführung der Gruppe Y.

11. Mischung enthaltend mindestens eine Verbindung nach einem oder mehreren der Ansprüche 1 bis 10 und mindestens eine weitere Verbindung.

12. Formulierung, insbesondere eine Lösung, eine Suspension oder eine Miniemulsion, enthaltend mindestens eine Verbindung nach einem oder mehreren der Ansprüche 1 bis 10 oder eine Mischung nach Anspruch 12 und ein oder mehrere Lösemittel.

13. Verwendung einer Verbindung nach einem oder mehreren der Ansprüche 1 bis 10 oder einer Mischung nach Anspruch 12 in einer elektronischen Vorrichtung.

14. Elektronische Vorrichtung enthaltend mindestens eine Verbindung nach einem oder mehreren der Ansprüche 1 bis 10 oder eine Mischung nach Anspruch 12, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

15. Elektronische Vorrichtung nach Anspruch 15, wobei es sich um eine organische Elektrolumineszenzvorrichtung handelt, **dadurch gekennzeichnet, dass** die Verbindung nach einem oder mehreren der Ansprüche 1 bis 10 als Matrixmaterial für fluoreszierende oder phosphoreszierende Emitter und/oder in einer elektronenblockierenden bzw. exzitonenblockierenden Schicht und/oder in einer Lochtransport- bzw. Lochinjektionsschicht eingesetzt wird.

## Claims

1. Compound of the formula (1 a) or (2a), 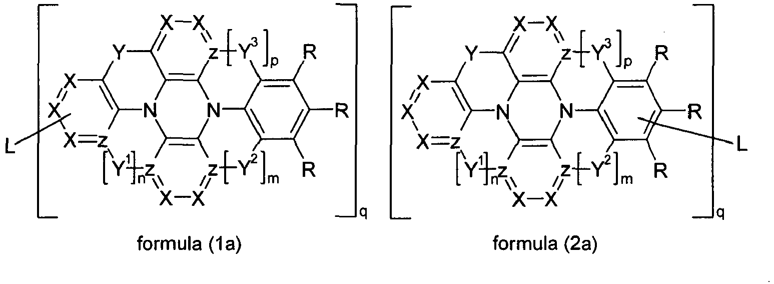 where the following applies to the symbols and indices used:
X is on each occurrence, identically or differently, CR or N; or X stands for C if a group L is bonded to this group X;
Y, Y¹, Y², Y³ are on each occurrence, identically or differently, a single bond or C(R')₂, O or S; with the proviso that Y and Y¹ do not simultaneously stand for a single bond and that Y² and Y³ do not simultaneously stand for a single bond;
Z is on each occurrence, identically or differently, CR or N; or Z stands for C if a group Y¹ or Y² or Y³ is bonded to this group Z;
L is, identically or differently, R if q = 1 or is a di-, tri-, tetra-, penta- or hexavalent straight-chain alkylene, alkylidene, alkyleneoxy or thioalkyleneoxy group having 1 to 40 C atoms or a branched or cyclic alkylene, alkylidene, alkyleneoxy or thioalkyleneoxy group having 3 to 40 C atoms or an alkenylene or alkynylene group having 2 to 40 C atoms, which may be substituted by in each case one or more radicals R², where in each case one or more non-adjacent CH₂ groups may be replaced by -R²C=CR²-, -C=C-, Si(R²)₂, C=O, C=NR², P(=O)R², S=O, SO₂, -O-, -S- or -CONR²- and where one or more H atoms may be replaced by D, F, Cl, Br, I, CN or NO₂, or a di-, tri-, tetra-, penta- or hexavalent aromatic ring system having 5 to 40 aromatic ring atoms, which may be substituted by one or more radicals R², or heteroaromatic ring system having 5 to 60 aromatic ring atoms, which contains, as heteroaryl groups, exclusively sulfur-containing or oxygen-containing heteroaryl groups and which may be substituted by one or more radicals R², or P(R²)₃₋ᵣ, P(=O)(R²)₃₋ᵣ, C(R²)₄₋ᵣ, Si(R²)₄₋ᵣ, N(Ar)₃₋ᵣ, where r stands for 2, 3 or 4, with the proviso that r is not greater than the maximum valence of L; or L is a chemical bond, in which case q = 2; the valence of the group L = q + 1 here;
n, m, p are on each occurrence, identically or differently, 0 or 1, where n = 0 or m = 0 or p = 0 means that the corresponding group Y¹ or Y² or Y³ respectively is not present and that a group R is bonded to the corresponding carbon atom instead of Y² or Y³;
q is 1, 2, 3, 4, 5 or 6;
R, R¹ are selected on each occurrence, identically or differently, from the group consisting of H, D, F, Cl, Br, I, CN, NO₂, C(=O)Ar, C(=O)R², P(=O)(Ar)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may kin each case be substituted by one or more radicals R², where one or more nonadjacent CH₂ groups may be replaced by R²C=CR², C=C, Si(R²)₂, C=O, C=NR², P(=O)(R²), SO, SO₂, NR², O, S or CONR² and where one or more H atoms may be replaced by D, F, Cl, Br, I, CN or NO₂, an aromatic ring system having 6 to 60 aromatic ring atoms, which may in each case be substituted by one or more radicals R², a heteroaromatic ring system having 5 to 60 aromatic ring atoms which contains, as heteroaryl groups, sulfur-containing or oxygen-containing heteroaryl groups and which may be substituted by one or more radicals R², an aryloxy or heteroaryloxy group having 5 to 60 aromatic ring atoms, which may be substituted by one or more radicals R², or an aralkyl or heteroaralkyl group having 5 to 60 aromatic ring atoms, which may be substituted by one or more radicals R², where two substituents R¹ which are bonded in the same group Y may optionally form a mono- or polycyclic, aliphatic, aromatic or heteroaromatic ring system with one another, which may be substituted by one or more radicals R²; furthermore, two adjacent radicals R may form a condensed-on benzo ring, which may be substituted by one or more radicals R²; the group R is not present in formula (2) if the group L is bonded to the corresponding carbon atom;
R² is selected on each occurrence, identically or differently, from the group consisting of H, D, F, Cl, Br, I, CN, NO₂, C(=O)Ar, C(=O)R³, P(=O)(Ar)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R³, where one or more non-adjacent CH₂ groups may be replaced by R³C=CR³, C=C, Si(R³)₂, C=O, C=NR³, P(=O)(R³), SO, SO₂, NR³, O, S or CONR³ and where one or more H atoms may be replaced by D, F, Cl, Br, I, CN or NO₂, an aromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³, or an aralkyl or heteroaralkyl group having 5 to 60 aromatic ring atoms;
Ar is on each occurrence, identically or differently, an aromatic or heteroaromatic ring system having 5-30 aromatic ring atoms, which may be substituted by one or more non-aromatic radicals R³; two radicals Ar which are bonded to the same P atom may also be bridged to one another by a single bond or a bridge selected from N(R³), C(R³)₂, O or S;
R³ is selected from the group consisting of H, D, F, CN, an aliphatic hydrocarbon radical having 1 to 20 C atoms or an aromatic ring system having 5 to 30 aromatic ring atoms, in which one or more H atoms may be replaced by D, F, Cl, Br, I or CN;
the following compounds are excluded from the invention:

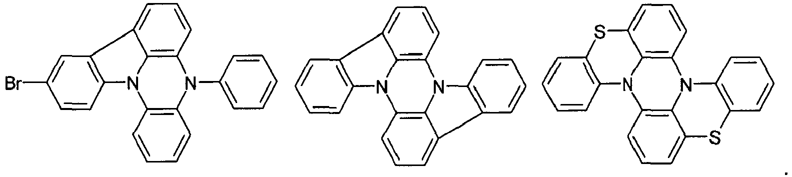

2. Compound according to Claim 1, **characterised in that**
X, Z are CR or N, with the proviso that a maximum of one of the groups X and Z per ring stands for N and the other groups X and Z in this ring stand for CR, or Z stands for C if a group Y¹ or Y² or Y³ is bonded to this Z, and X stands for C if a group L is bonded to this X.

3. Compound according to one or more of Claims 1 and 2, **characterised in that** the compound is selected from the compounds of the formulae (1 b) and (2b), 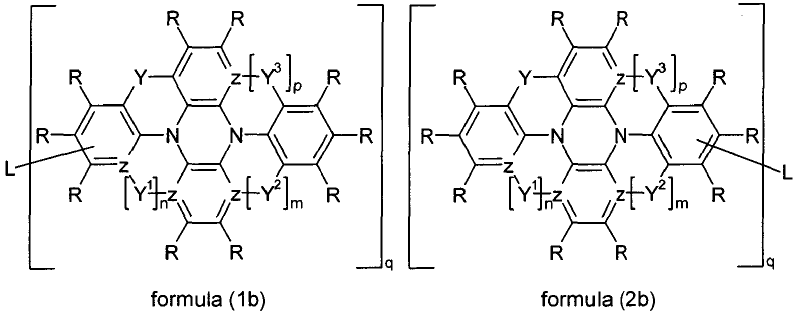 where the following applies to the symbols used:
Y, Y¹, Y², Y³ are, identically or differently on each occurrence, a single bond, C(R')₂, O or S, in particular a single bond or C(R¹)₂;
Z is CR, or Z is C if a group Y¹ or Y² or Y³ is bonded to this Z;
and the other symbols and indices used have the meanings given in Claim 1; the radical R is not present here if a group L is bonded in this position.

4. Compound according to one or more of Claims 1 to 3, **characterised in that** the compound is selected from the compounds of the formulae (3) to (8), 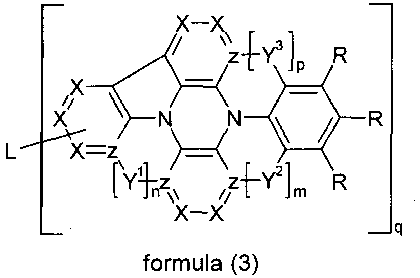 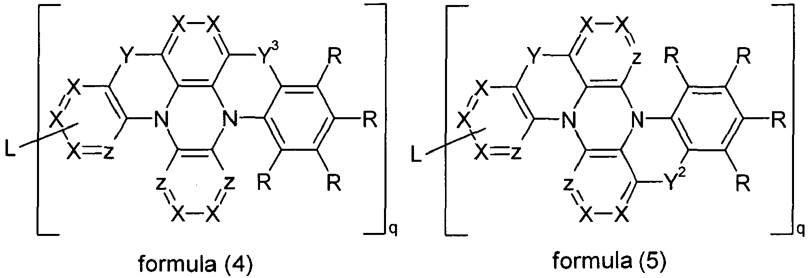 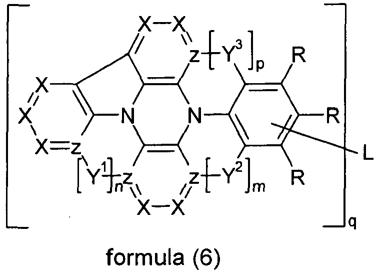 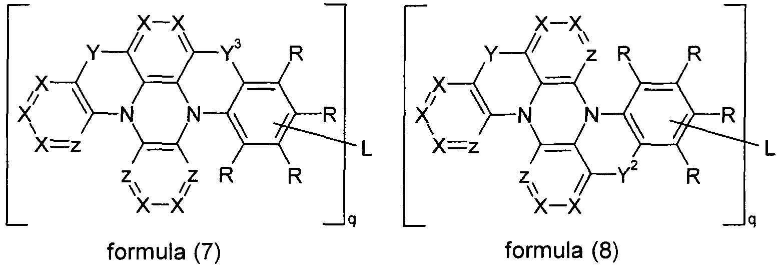 where the symbols and indices used have the meanings given in Claim 1 and the group R is not present if a group L is bonded in this position.

5. Compound according to one or more of Claims 1 to 4, **characterised in that** the compound is selected from the compounds of the formulae (3a), (4a) and (5a), 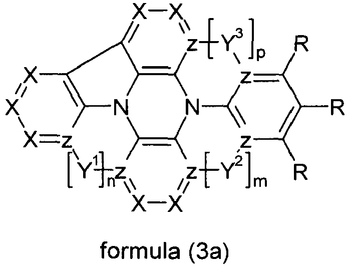 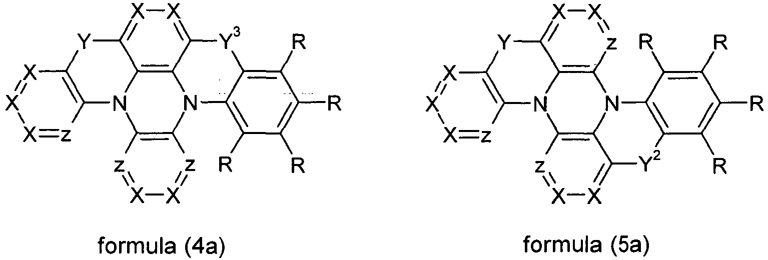 where the symbols and indices used have the meanings given in Claim 1.

6. Compound according to one or more of Claims 1 to 5, **characterised in that** the compound of the formula (3) is selected from the compounds of the formulae (19) to (23), 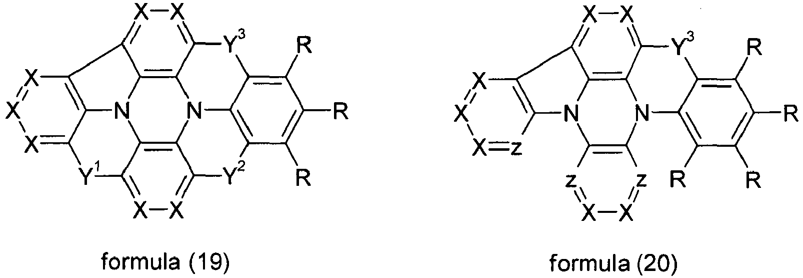 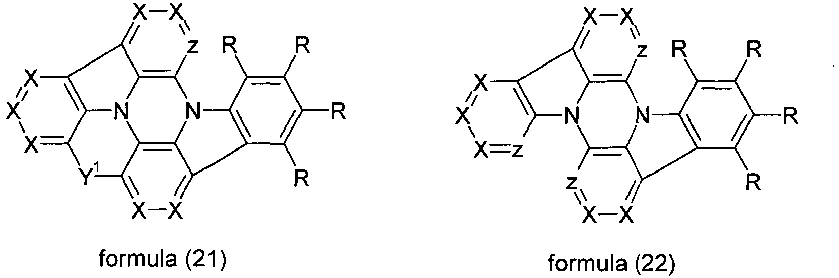 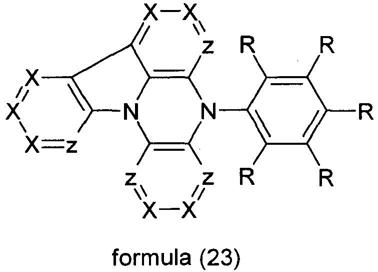 where the symbols and indices used have the meanings given in Claim 1.

7. Compounds according to one or more of Claims 1 to 6, **characterised in that** the compounds are selected from the compounds of the formulae (19a) to (23a), (24) and (25), 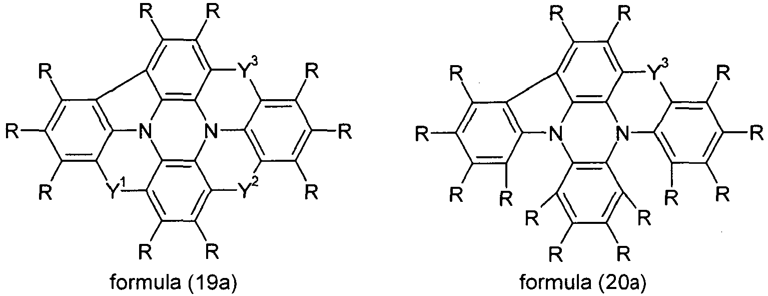 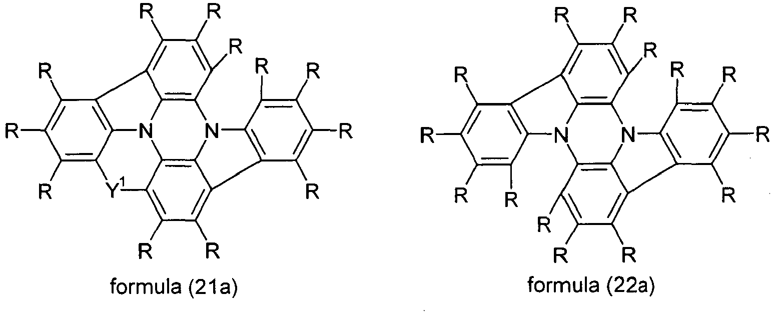 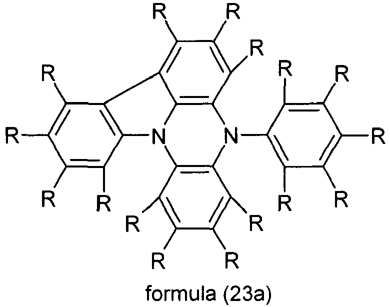 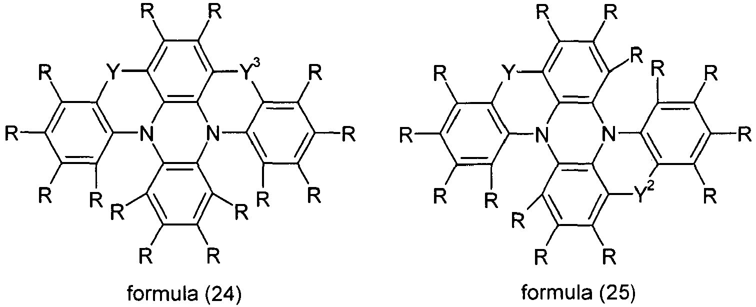 where the symbols used have the meanings given under Claim 1.

8. Compound according to one or more of Claims 1 to 7, **characterised in that** the compound is selected from the compounds of the formulae (19b) to (23b), (24a) and (25a), 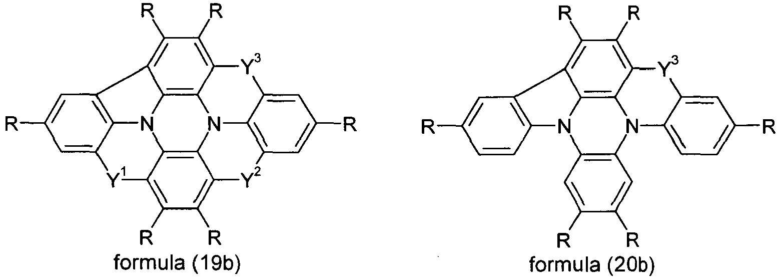 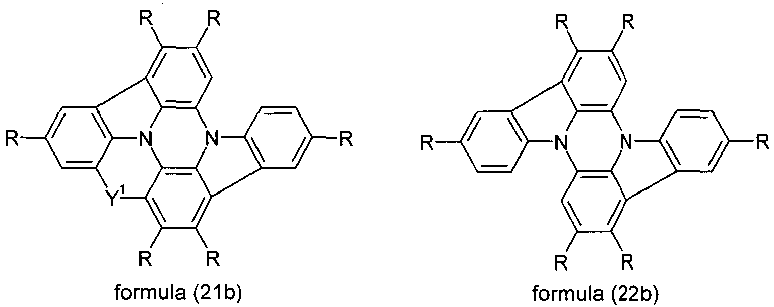 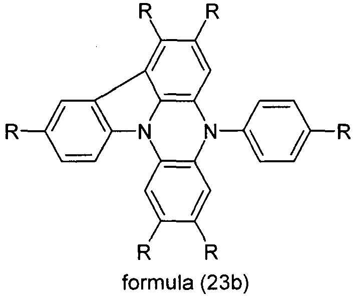 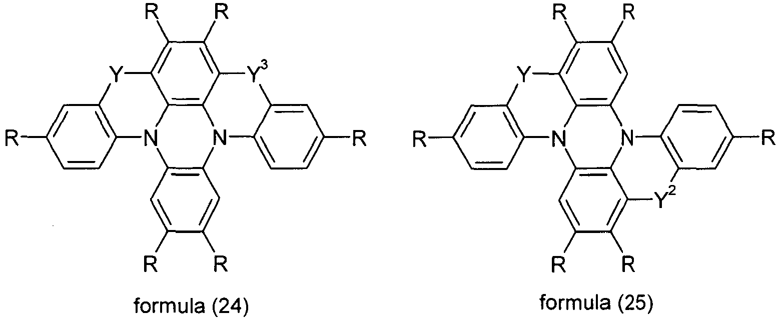 where the symbols used have the meanings given in Claim 1.

9. Compound according to one or more of Claims 1 to 8, **characterised in that**, if R stands for an aromatic or heteroaromatic ring system, R is selected from the groups of the formulae (26) to (58), 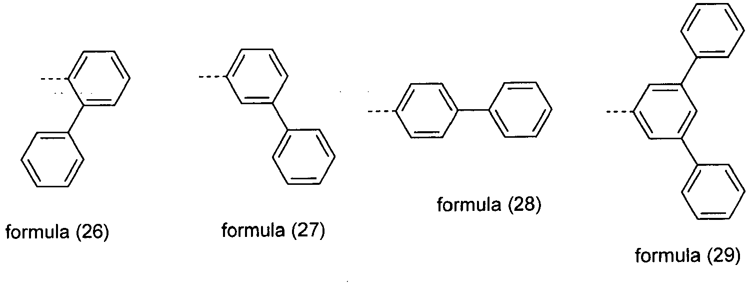 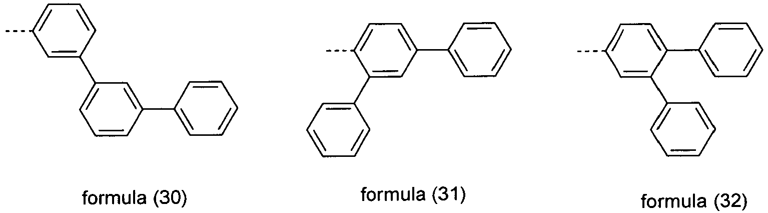 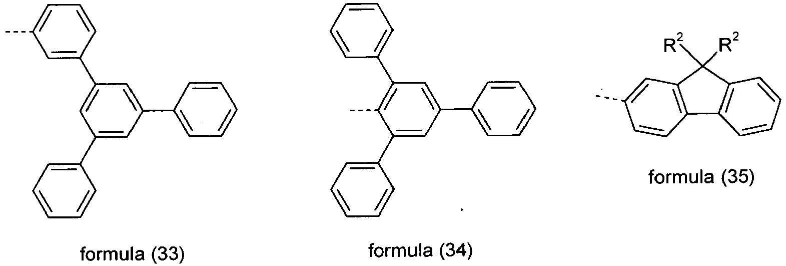 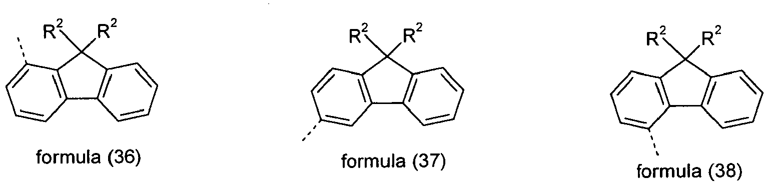 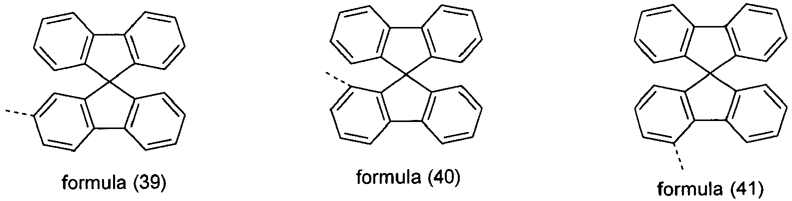 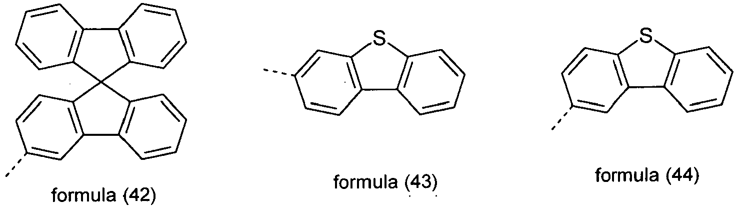 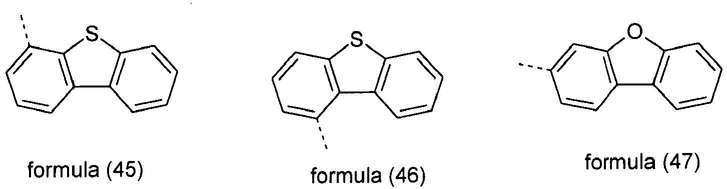 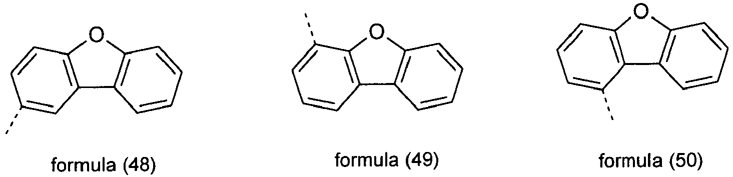 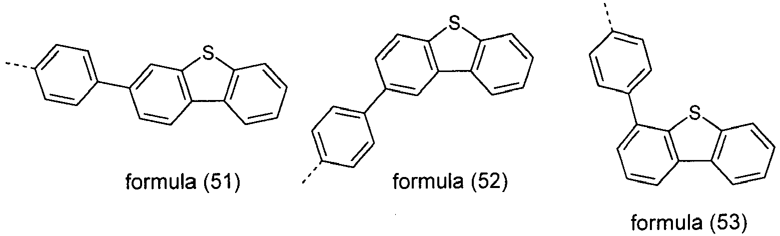 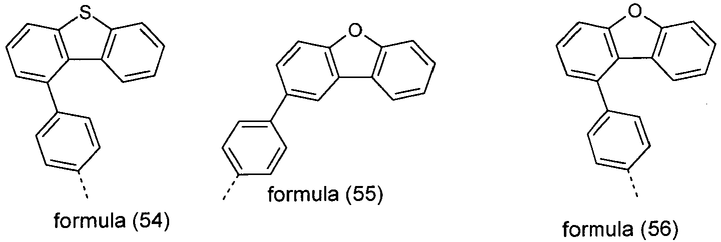 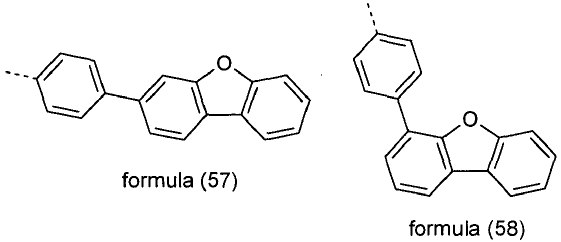 where the dashed bond indicates the bonding to the basic structure and the groups may be substituted by one or more radicals R².

10. Process for the preparation of a compound according to one or more of Claims 1 to 9, comprising the reaction steps:
a) synthesis of a compound of the formula (1 a) or (1 b) which does not yet contain a bridge Y; and
b) introduction of the group Y.

11. Mixture comprising at least one compound according to one or more of Claims 1 to 9 and at least one further compound.

12. Formulation, in particular a solution, a suspension or a miniemulsion, comprising at least one compound according to one or more of Claims 1 to 9 or a mixture according to Claim 11 and one or more solvents.

13. Use of a compound according to one or more of Claims 1 to 9 or a mixture according to Claim 11 in an electronic device.

14. Electronic device containing at least one compound according to one or more of Claims 1 to 9 or a mixture according to Claim 11, preferably selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and "organic plasmon emitting devices".

15. Electronic device according to Claim 14, which is an organic electroluminescent device, **characterised in that** the compound according to one or more of Claims 1 to 9 is employed as matrix material for fluorescent or phosphorescent emitters and/or in an electron-blocking or exciton-blocking layer and/or in a hole-transport or hole-injection layer.

## Revendications

1. Composé de la formule (1 a) ou (2a) : 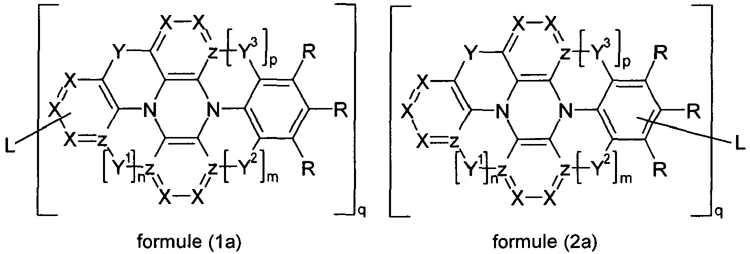 dans lesquelles ce qui suit s'applique aux symboles et indices utilisés :
X est pour chaque occurrence, de manière identique ou différente, CR ou N ; ou X représente C si un groupe L est lié à ce groupe X ;
Y, Y¹, Y², Y³ sont pour chaque occurrence, de manière identique ou différente, une liaison simple ou C(R')₂, O ou S ; étant entendu que Y et Y¹ ne représentent pas simultanément une liaison simple et que Y² et Y³ ne représentent pas simultanément une liaison simple ;
Z est pour chaque occurrence, de manière identique ou différente, CR ou N ; ou Z représente C si un groupe Y¹ ou Y² ou Y³ est lié à ce groupe Z ;
L est, de manière identique ou différente, R si q = 1 ou est un groupe alkylène, alkylidène, alkylèneoxy ou thioalkylèneoxy en chaîne droite divalent, trivalent, tétravalent, pentavalent ou hexavalent qui comporte 1 à 40 atome(s) de C ou un groupe alkylène, alkylidène, alkylèneoxy ou thioalkylèneoxy ramifié ou cyclique qui comporte 3 à 40 atomes de C ou un groupe alkénylène ou alkynylène qui comporte 2 à 40 atomes de C, lequel groupe peut être substitué par, dans chaque cas, un radical ou plusieurs radicaux R², où, dans chaque cas, un ou plusieurs groupe(s) CH₂ non adjacents peut/peuvent être remplacé(s) par -R²C=CR²-, -C=C-, Si(R²)₂, C=O, C=NR², P(=O)R², S=O, SO₂, -O-, -S- ou -CONR²- et où un ou plusieurs atome(s) de H peut/peuvent être remplacé(s) par D, F, Cl, Br, I, CN ou NO₂, ou un système de cycle aromatique divalent, trivalent, tétravalent, pentavalent ou hexavalent qui comporte 5 à 40 atomes de cycle aromatique, lequel système peut être substitué par un radical ou plusieurs radicaux R², ou un système de cycle hétéroaromatique qui comporte 5 à 60 atomes de cycle aromatique, lequel système contient, en tant que groupes hétéroaryle, de manière exclusive des groupes hétéroaryle qui contiennent du soufre ou qui contiennent de l'oxygène et qui peuvent être substitués par un radical ou plusieurs radicaux R², ou P(R²)₃₋ᵣ, P(=O)(R²)₃₋ᵣ, C(R²)₄₋ᵣ, Si(R²)₄₋ᵣ, N(Ar)₃₋ᵣ, où r représente 2, 3 ou 4, étant entendu que r n'est pas supérieur à la valence maximum de L ; ou L est une liaison chimique, auquel cas q = 2 ; la valence du groupe L = q + 1 ici ;
n, m, p sont pour chaque occurrence, de manière identique ou différente, 0 ou 1, où n = 0 ou m = 0 ou p = 0 signifie que le groupe correspondant Y¹ ou Y² ou Y³, de manière respective, n'est pas présent et qu'un groupe R est lié à l'atome de carbone correspondant en lieu et place de Y² ou de Y³ ;
q est 1, 2, 3, 4, 5 ou 6 ;
R, R¹ sont sélectionnés pour chaque occurrence, de manière identique ou différente, parmi le groupe qui est constitué par H, D, F, Cl, Br, I, CN, NO₂, C(=O)Ar, C(=O)R², P(=O)(Ar)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut dans chaque cas être substitué par un radical ou plusieurs radicaux R², où un ou plusieurs groupe(s) CH₂ non adjacents peut/peuvent être remplacé(s) par R²C=CR², C≡C, Si(R²)₂, C=O, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, un système de cycle aromatique qui comporte 6 à 60 atomes de cycle aromatique, lequel peut dans chaque cas être substitué par un radical ou plusieurs radicaux R², un système de cycle hétéroaromatique qui comporte 5 à 60 atomes de cycle aromatique, lequel contient, en tant que groupes hétéroaryle, des groupes hétéroaryle qui contiennent du soufre ou qui contiennent de l'oxygène et lequel peut être substitué par un radical ou plusieurs radicaux R², un groupe aryloxy ou hétéroaryloxy qui comporte 5 à 60 atomes de cycle aromatique, lequel peut être substitué par un radical ou plusieurs radicaux R², ou un groupe aralkyle ou hétéroaralkyle qui comporte 5 à 60 atomes de cycle aromatique, lequel peut être substitué par un radical ou plusieurs radicaux R², où deux substituants R¹ qui sont liés dans le même groupe Y peuvent en option former un système de cycle aliphatique, aromatique ou hétéroaromatique monocyclique ou polycyclique, l'un avec l'autre, lequel peut être substitué par un radical ou plusieurs radicaux R² ; qui plus est, deux radicaux R adjacents peuvent former un cycle benzo avec condensation, lequel peut être substitué par un radical ou plusieurs radicaux R², le groupe R n'est pas présent dans la formule (2) si le groupe L est lié à l'atome de carbone correspondant ;
R² est sélectionné pour chaque occurrence, de manière identique ou différente, parmi le groupe qui est constitué par H, D, F, Cl, Br, I, CN, NO₂, C(=O)Ar, C(=O)R³, P(=O)(Ar)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut dans chaque cas être substitué par un radical ou plusieurs radicaux R³, où un ou plusieurs groupe(s) CH₂ non adjacents peut/peuvent être remplacé(s) par R³C=CR³, C=C, Si(R³)₂, C=O, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, un système de cycle aromatique qui comporte 5 à 60 atomes de cycle aromatique, lequel peut dans chaque cas être substitué par un radical ou plusieurs radicaux R³, un groupe aryloxy ou hétéroaryloxy qui comporte 5 à 60 atomes de cycle aromatique, lequel peut être substitué par un radical ou plusieurs radicaux R³, ou un groupe aralkyle ou hétéroaralkyle qui comporte 5 à 60 atomes de cycle aromatique ;
Ar est pour chaque occurrence, de manière identique ou différente, un système de cycle aromatique ou hétéroaromatique qui comporte 5 à 30 atomes de cycle aromatique, lequel peut être substitué par un radical ou plusieurs radicaux non aromatique(s) R³ ; deux radicaux Ar qui sont liés au même atome de P peuvent également être pontés l'un à l'autre au moyen d'une liaison simple ou d'un pont qui est sélectionné parmi N(R³), C(R³)₂, O ou S ;
R³ est sélectionné parmi le groupe qui est constitué par H, D, F, CN, un radical hydrocarbone aliphatique qui comporte 1 à 20 atome(s) de C ou un système de cycle aromatique qui comporte 5 à 30 atomes de cycle aromatique, où un ou plusieurs atome(s) de H peut/peuvent être remplacé(s) par D, F, Cl, Br, I ou CN ;
les composés qui suivent sont exclus de l'invention :

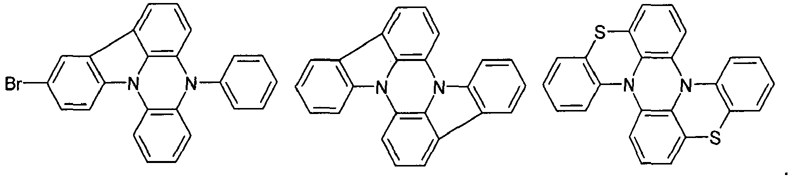

2. Composé selon la revendication 1, **caractérisé en ce que** :
X, Z sont CR ou N, étant entendu qu'un maximum d'un des groupes X et Z par cycle représente N et les autres groupes X et Z dans ce cycle représentent CR, ou Z représente C si un groupe Y¹ ou Y² ou Y³ est lié à ce Z, et X représente C si un groupe L est lié à ce X.

3. Composé selon une ou plusieurs des revendications 1 et 2, **caractérisé en ce que** le composé est sélectionné parmi les composés des formules (1 b) et (2b) : 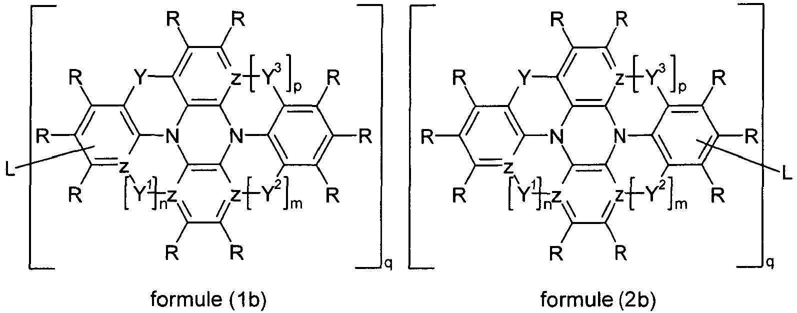 dans lesquelles ce qui suit s'applique aux symboles utilisés :
Y, Y¹, Y², Y³ sont, de manière identique ou différente pour chaque occurrence, une liaison simple, C(R')₂, O ou S, en particulier une liaison simple ou C(R¹)₂ ;
Z est CR, ou Z est C si un groupe Y¹ ou Y² ou Y³ est lié à ce Z ;
et les autres symboles et indices qui sont utilisés présentent les significations qui sont données dans la revendication 1 ; le radical R n'est pas présent ici si un groupe L est lié au niveau de cette position.

4. Composé selon une ou plusieurs des revendications 1 à 3, **caractérisé en ce que** le composé est sélectionné parmi les composés des formules (3) à (8) : 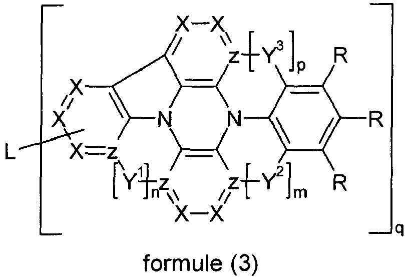 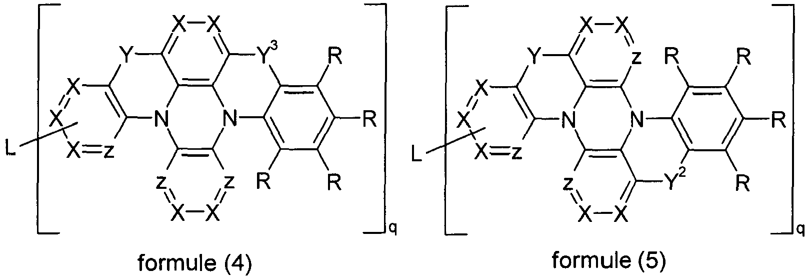 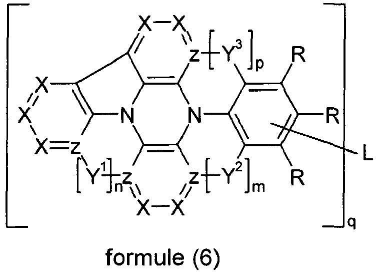 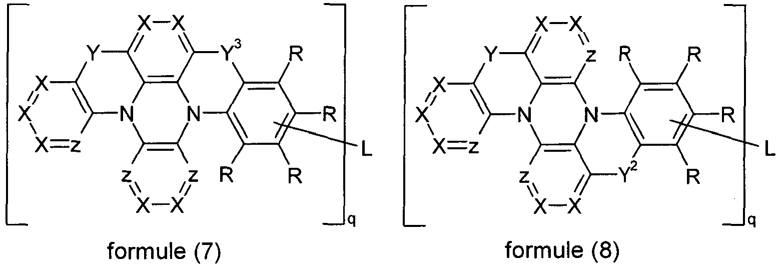 dans lesquelles les symboles et indices qui sont utilisés présentent les significations qui sont données dans la revendication 1 et le groupe R n'est pas présent si un groupe L est lié au niveau de cette position.

5. Composé selon une ou plusieurs des revendications 1 à 4, **caractérisé en ce que** le composé est sélectionné parmi les composés des formules (3a), (4a) et (5a) : 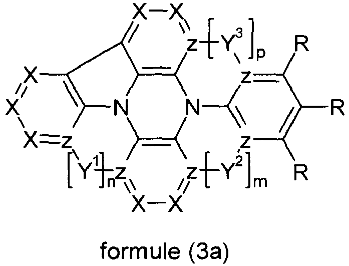 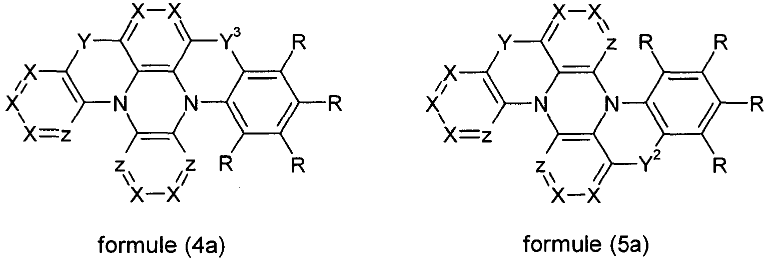 dans lesquelles les symboles et indices qui sont utilisés présentent les significations qui sont données dans la revendication 1.

6. Composé selon une ou plusieurs des revendications 1 à 5, **caractérisé en ce que** le composé de la formule (3) est sélectionné parmi les composés des formules (19) à (23) : 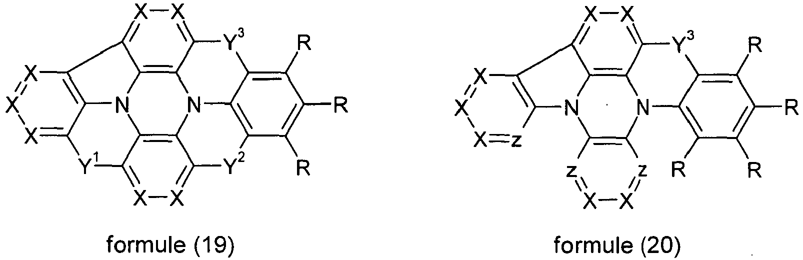 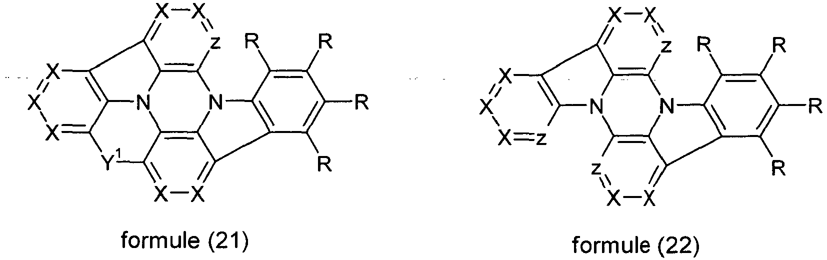 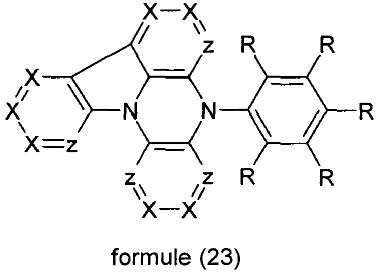 dans lesquelles les symboles et indices qui sont utilisés présentent les significations qui sont données dans la revendication 1.

7. Composés selon une ou plusieurs des revendications 1 à 6, **caractérisés en ce que** les composés sont sélectionnés parmi les composés des formules (19a) à (23a), (24) et (25) : 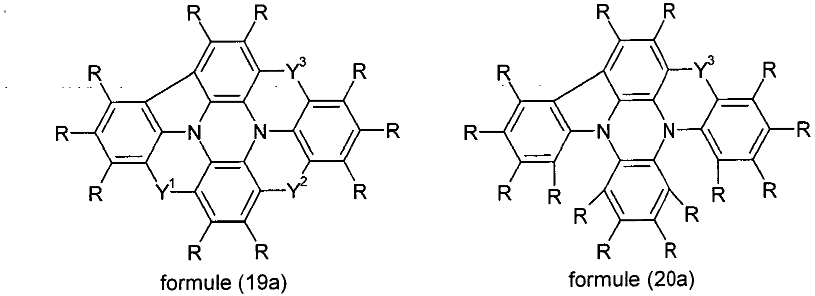 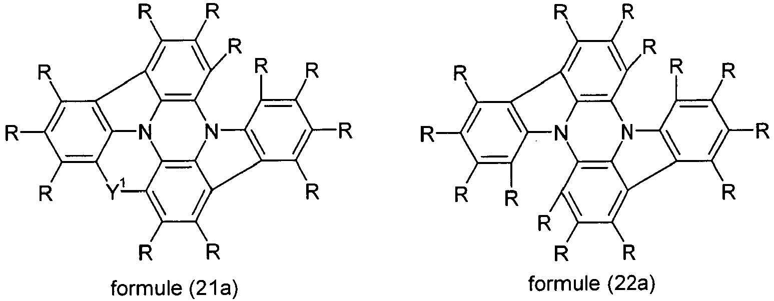 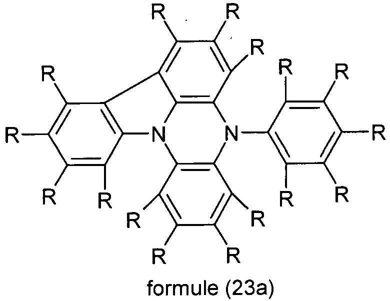 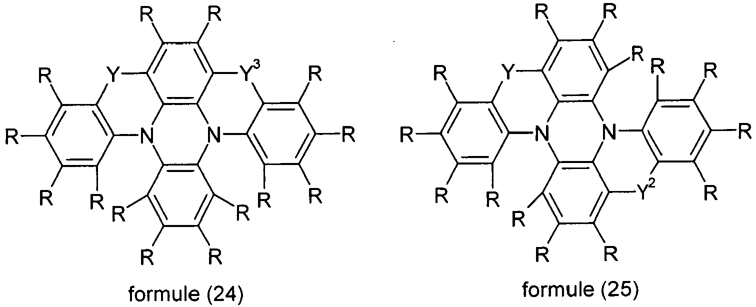 dans lesquelles les symboles qui sont utilisés présentent les significations qui sont données sous la revendication 1.

8. Composé selon une ou plusieurs des revendications 1 à 7, **caractérisé en ce que** le composé est sélectionné parmi les composés des formules (19b) à (23b), (24a) et (25a) : 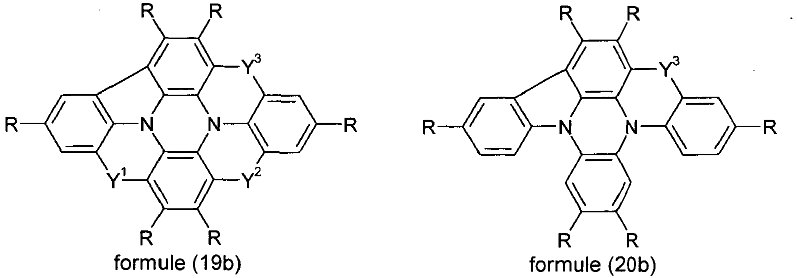 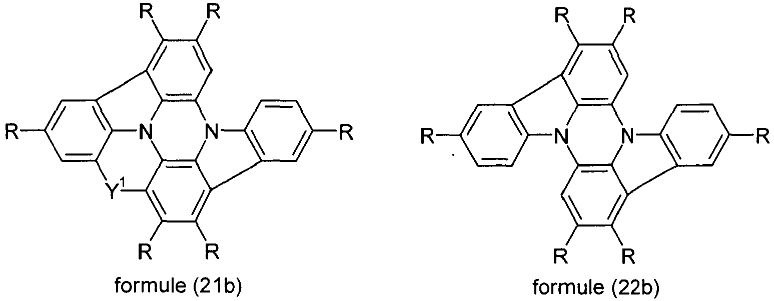 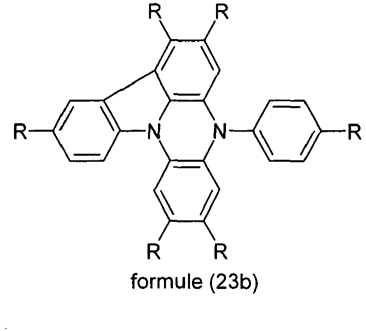 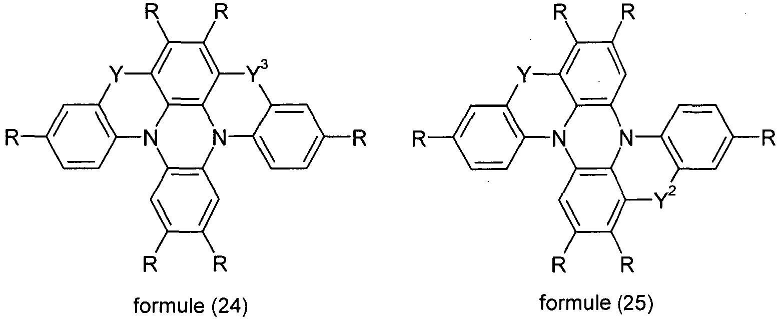 dans lesquelles les symboles qui sont utilisés présentent les significations qui sont données dans la revendication 1.

9. Composé selon une ou plusieurs des revendications 1 à 8, **caractérisé en ce que**, si R représente un système de cycle aromatique ou hétéroaromatique, R est sélectionné parmi les groupes des formules (26) à (58) : 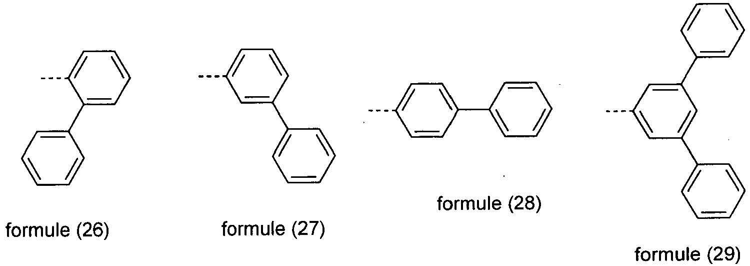 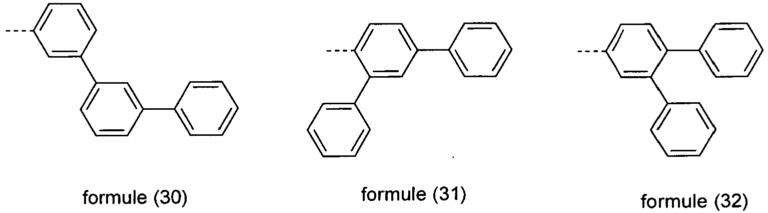 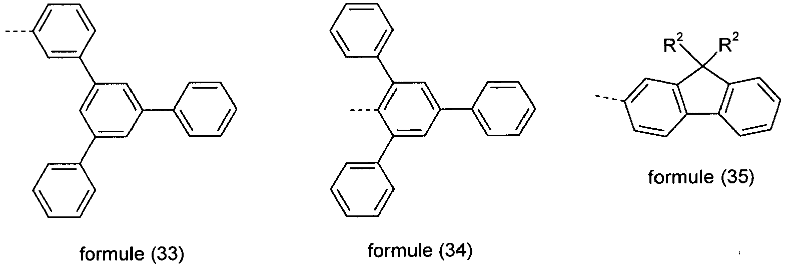 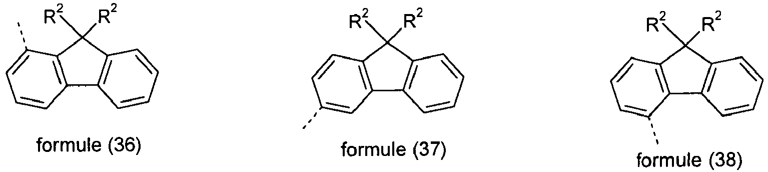 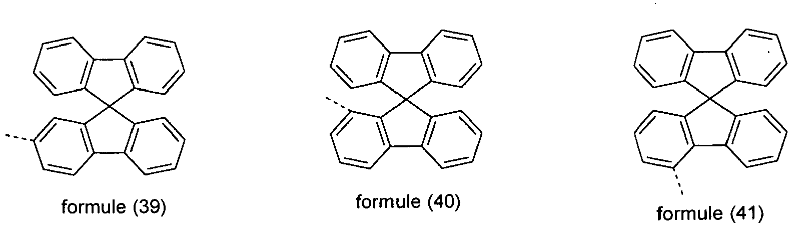 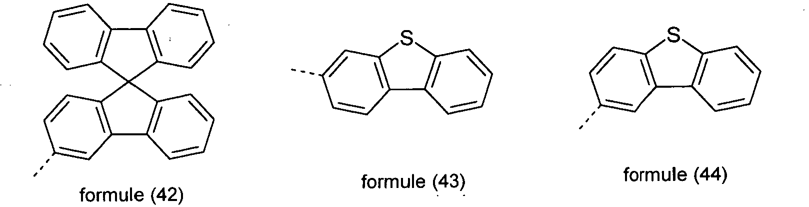 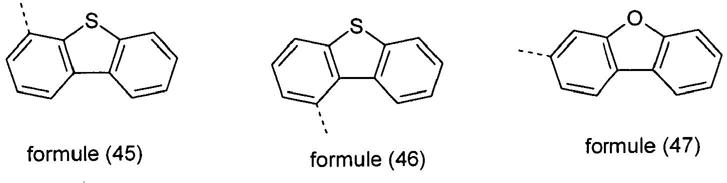 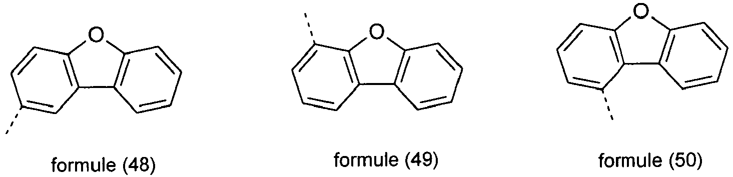 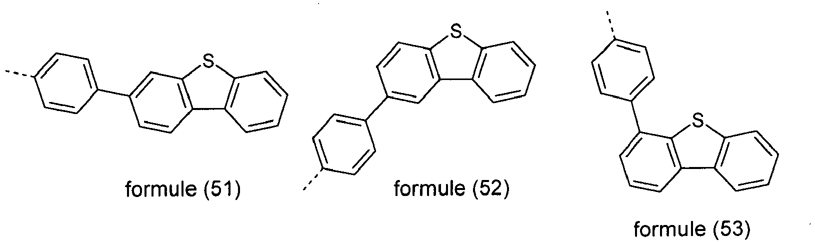 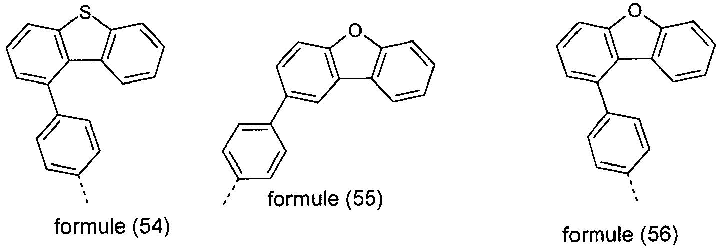 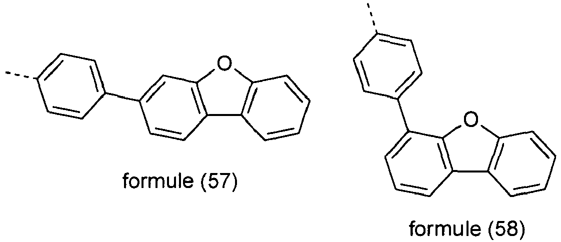 dans lesquelles la liaison en pointillés indique la liaison sur la structure de base et les groupes peuvent être substitués par un radical ou plusieurs radicaux R².

10. Procédé pour la préparation d'un composé selon une ou plusieurs des revendications 1 à 9, comprenant les étapes de réaction qui suivent :
a) la synthèse d'un composé de la formule (1 a) ou (1 b) qui ne contient pas encore un pont Y ; et
b) l'introduction du groupe Y.

11. Mélange comprenant au moins un composé selon une ou plusieurs des revendications 1 à 9 et au moins un autre composé.

12. Formulation, en particulier une solution, une suspension ou une mini-émulsion, comprenant au moins un composé selon une ou plusieurs des revendications 1 à 9 ou mélange selon la revendication 11 et un ou plusieurs solvant(s).

13. Utilisation d'un composé selon une ou plusieurs des revendications 1 à 9 ou d'un mélange selon la revendication 11 dans un dispositif électronique.

14. Dispositif électronique contenant au moins un composé selon une ou plusieurs des revendications 1 à 9 ou un mélange selon la revendication11, de façon préférable sélectionné parmi le groupe qui est constitué par des dispositifs électroluminescents organiques, des circuits intégrés organiques, des transistors à effet de champ organiques, des transistors à film mince organiques, des transistors à émission de lumière organiques, des cellules solaires organiques, des cellules solaires sensibilisées par colorant organiques, des détecteurs optiques organiques, des photorécepteurs organiques, des dispositifs à extinction de champ organiques, des cellules électrochimiques à émission de lumière, des diodes laser organiques et des "dispositifs à émission de plasmons organiques".

15. Dispositif électronique selon la revendication 14, lequel est un dispositif électroluminescent organique, **caractérisé en ce que** le composé selon une ou plusieurs des revendications 1 à 9 est utilisé en tant que matériau de matrice pour des émetteurs fluorescents ou phosphorescents et/ou dans une couche de blocage d'électrons ou de blocage d'excitons et/ou dans une couche de transport de trous ou d'injection de trous.
